# EUROPEAN PATENT SPECIFICATION

(11) **EP 2 908 914 B1**
(45) Date of publication and mention of the grant of the patent: **18.04.2018**
(21) Application number: 12788627.3
(22) Date of filing: 17.10.2012
(51) Int. Cl.: A61K 38/00, C07K 16/28, C07K 14/47, A61K 39/00, C07K 16/00, C07K 16/46

(54) **IMMUNOMODULATORY PROTEINS**
IMMUNMODULATORISCHE PROTEINE
PROTÉINES IMMUNOMODULATRICES

(43) Date of publication of application: 26.08.2015
(73) Proprietor: Liverpool School of Tropical Medicine, Liverpool L3 5QA (GB)
(72) Inventor: PLEASS, Richard John, Liverpool L3 5QA (GB)
(74) Representative: HGF Limited
(86) International application number: PCT/GB2012/052561
(87) International publication number: WO 2014/060712

(56) References cited:
- WO-A1-2011/073692
- WO-A2-2008/151088
- WO-A2-2012/016073
- SORENSEN V ET AL: "Effect of the IgM and IgA secretory tailpieces on polymerization and secretion of IgM and IgG", THE JOURNAL OF IMMUNOLOGY, THE AMERICAN ASSOCIATION OF IMMUNOLOGISTS, US, vol. 156, no. 8, 15 April 1996 (1996-04-15), pages 2858-2865, XP002111658, ISSN: 0022-1767
- AJAY JAIN ET AL: "Fully recombinant IgG2a Fc multimers (stradomers) effectively treat collagen-induced arthritis and prevent idiopathic thrombocytopenic purpura in mice", ARTHRITIS RESEARCH & THERAPY, vol. 14, no. 4, 1 August 2012 (2012-08-01) , pages R192-R192, XP055070050, ISSN: 1478-6354, DOI: 10.1136/ard.54.5.382
- DAVID M WHITE ET AL: "Design and expression of polymeric immunoglobulin fusion proteins: a strategy for targeting low-affinity Fcgamma receptors", PROTEIN EXPRESSION AND PURIFICATION, ACADEMIC PRESS, SAN DIEGO, CA, vol. 3, 21 April 2001 (2001-04-21), pages 446-455, XP008126475, ISSN: 1046-5928, DOI: 10.1006/PREP.2001.1406
- MEKHAIEL DAVID N A ET AL: "Polymeric human Fc-fusion proteins with modified effector functions", SCIENTIFIC REPORTS, vol. 1, October 2011 (2011-10), XP002708372, cited in the application

### SEQUENCE LISTING

<110> Liverpool School of Tropical Medicine
   <120> Immunomodulatory proteins
<130> LIVBM/P50745PC
<160> 14
<170> SeqWin99
<210> 1
   <211> 64
   <212> DNA
   <213> Artificial Sequence
<220>
   <223> primer 1
<400> 1
   ctaggacccc ccctgtacaa cgtgtccctg gtcatgtccg acacagctgg cacctgctac 60
   tgag 64
<210> 2
   <211> 64
   <212> DNA
   <213> Artificial Sequence
<220>
   <223> primer 2
<400> 2
   ctagctcagt agcaggtgcc agctgtgtcg gacatgacca gggacacgtt gtacaggggg 60
   ggtc 64
<210> 3
   <211> 36
   <212> DNA
   <213> Artificial Sequence
<220>
   <223> primer 3
<400> 3
   ctgtctccgg gtaaattagt cctaggaccc cccctg 36
<210> 4
   <211> 36
   <212> DNA
   <213> Artificial Sequence
<220>
   <223> primer 4
<400> 4
   cagggggggt cctaggacta atttacccgg agacag 36
<210> 5
   <211> 48
   <212> DNA
   <213> Artificial Sequence
<220>
   <223> primer 5
<400> 5
   gtggtcagcg tcctcaccgt ctgcctccag gactggctga atggcaag 48
<210> 6
   <211> 48
   <212> DNA
   <213> Artificial Sequence
<220>
   <223> primer 6
<400> 6
   cttgccattc agccagtcct ggaggcagac ggtgaggacg ctgaccac 48
<210> 7
   <211> 810
   <212> DNA
   <213> Artificial Sequence
<220>
   <223> Fc protein monomer unit coding sequence
<400> 7
   atgtacagga tgcaactcct gtcttgcatt gcactaagtc ttgcacttgt cacgaattcc   60
   gacaaaactc acacatgccc accgtgccca gcacctgaac tcctgggggg accgtcagtc   120
   ttcctcttcc ccccaaaacc caaggacacc ctcatgatct cccggacccc tgaggtcaca   180
   tgcgtggtgg tggacgtgag ccacgaagac cctgaggtca agttcaactg gtacgtggac   240
   ggcgtggagg tgcataatgc caagacaaag ccgcgggagg agcagtacaa cagcacgtac   300
   cgtgtggtca gcgtcctcac cgtctgcctc caggactggc tgaatggcaa ggagtacaag   360
   tgcaaggtct ccaacaaagc cctcccagcc cccatcgaga aaaccatctc caaagccaaa   420
   gggcagcccc gagaaccaca ggtgtacacc ctgcccccat cccgggagga gatgaccaag   480
   aaccaggtca gcctgacctg cctggtcaaa ggcttctatc ccagcgacat cgccgtggag   540
   tgggagagca atgggcagcc ggagaacaac tacaagacca cgcctcccgt gctggactcc   600
   gacggctcct tcttcctcta cagcaagctc accgtggaca agagcaggtg gcagcagggg   660
   aacgtcttct catgctccgt gatgcacgag gctctgcaca accactacac gcagaagagc   720
   ctctccctgt ctccgggtaa attagtccta ggaccccccc tgtacaacgt gtccctggtc   780
   atgtccgaca cagctggcac ctgctactga                                    810
<210> 8
   <211> 269
   <212> PRT
   <213> Artificial Sequence
<220>
   <223> Fc protein monomer unit protein sequence
<400> 8
   Met Tyr Arg Met Gln Leu Leu Ser Cys Ile Ala Leu Ser Leu Ala Leu
   1               5                   10                  15
   Val Thr Asn Ser Asp Lys Thr His Thr Cys Pro Pro Cys Pro Ala Pro
               20                  25                  30
   Glu Leu Leu Gly Gly Pro Ser Val Phe Leu Phe Pro Pro Lys Pro Lys
           35                  40                  45
   Asp Thr Leu Met Ile Ser Arg Thr Pro Glu Val Thr Cys Val Val Val
       50                  55                  60
   Asp Val Ser His Glu Asp Pro Glu Val Lys Phe Asn Trp Tyr Val Asp
   65                  70                  75                  80
   Gly Val Glu Val His Asn Ala Lys Thr Lys Pro Arg Glu Glu Gln Tyr
                   85                  90                  95
   Asn Ser Thr Tyr Arg Val Val Ser Val Leu Thr Val Cys Leu Gln Asp
               100                 105                 110
   Trp Leu Asn Gly Lys Glu Tyr Lys Cys Lys Val Ser Asn Lys Ala Leu
           115                 120                 125
   Pro Ala Pro Ile Glu Lys Thr Ile Ser Lys Ala Lys Gly Gln Pro Arg
       130                 135                 140
   Glu Pro Gln Val Tyr Thr Leu Pro Pro Ser Arg Glu Glu Met Thr Lys
   145                 150                 155                 160
   Asn Gln Val Ser Leu Thr Cys Leu Val Lys Gly Phe Tyr Pro Ser Asp
                   165                 170                 175
   Ile Ala Val Glu Trp Glu Ser Asn Gly Gln Pro Glu Asn Asn Tyr Lys
               180                 185                 190
   Thr Thr Pro Pro Val Leu Asp Ser Asp Gly Ser Phe Phe Leu Tyr Ser
           195                 200                 205
   Lys Leu Thr Val Asp Lys Ser Arg Trp Gln Gln Gly Asn Val Phe Ser
       210                 215                 220
   Cys Ser Val Met His Glu Ala Leu His Asn His Tyr Thr Gln Lys Ser
   225                 230                 235                 240
   Leu Ser Leu Ser Pro Gly Lys Leu Val Leu Gly Pro Pro Leu Tyr Asn
                   245                 250                 255
   Val Ser Leu Val Met Ser Asp Thr Ala Gly Thr Cys Tyr
               260                 265
<210> 9
   <211> 4
   <212> PRT
   <213> Artificial Sequence
<220>
   <223> Ig linker
<220>
   <221> Ig linker
<400> 9
   Leu Val Leu Gly
   1
<210> 10
   <211> 18
   <212> PRT
   <213> Homo Sapiens
<220>
   <221> Tailpiece region of human IgM
<400> 10
   Pro Thr Leu Tyr Asn Val Ser Leu Val Met Ser Asp Thr Ala Gly Thr
   1 5 10 15
   Cys Tyr
<210> 11
   <211> 18
   <212> PRT
   <213> Homo sapiens
<220>
   <221> Modified tailpiece region of human IgM
<400> 11
   Pro Pro Leu Tyr Asn Val Ser Leu Val Met Ser Asp Thr Ala Gly Thr
   1 5 10 15
   Cys Tyr
<210> 12
   <211> 20
   <212> PRT
   <213> Mus musculus
<220>
   <221> IgM tailpiece sequence
<400> 12
   Gly Lys Phe Thr Leu Tyr Asn Val Ser Leu Ile Met Ser Asp Thr Gly
   1 5 10 15
   Gly Thr Cys Tyr
   20
<210> 13
   <211> 18
   <212> PRT
   <213> Homo sapiens
<220>
   <221> Tailpiece region of human IgA
<400> 13
   Pro Thr His Val Asn Val Ser Val Val Met Ala Gln Val Asp Gly Thr
   1 5 10 15
   Cys Tyr
<210> 14
   <211> 7
   <212> PRT
   <213> Homo sapiens
<220>
   <221> Fc receptor CH2 domain
<400> 14
   Glu Leu Leu Gly Gly Pro Ser
   1               5

## Description

### FIELD OF THE INVENTION

The invention relates to engineered proteins having an immunomodulatory function, and their medical uses for treatment of autoimmune or inflammatory diseases. In particular, the engineered proteins may be used as replacements for intravenous immunoglobulin (IVIG).

### BACKGROUND OF THE INVENTION

Autoimmune and inflammatory diseases are responsible for substantial morbidity and mortality. In 2003, autoimmune diseases were the sixth most frequent underlying cause of death in all age groups below 75 years. One important treatment modality is intravenous immunoglobulin (IVIG) or IgG. Immune globulin products from human plasma were first developed to treat immune deficiencies. However, seventy percent of prescribed IVIG is now used for the treatment of autoimmune or inflammatory conditions. The worldwide consumption of IVIG increased from 300kg per year in 1980 to 100 tonnes per year in 2010. IVIG is derived from the pooled plasma of -3,000 anonymous donors, according to a lengthy and expensive manufacturing process. The need for extensive screening of donors and donated plasma for viruses contributes to the high cost. Given the increasing demand, and the strict regulation of IVIG production, shortages of IVIG can occur. IVIG preparations may be subject to inadequate sterility, the presence of impurities and lot-to-lot variation. They may vary greatly in their immunoglobulin A content, and IgA can cause allergic or anaphylactic reactions in IgA-deficient recipients, making them unsuitable for some patients. Very large doses of IVIG have to be given to patients, typically 2 g per kg bodyweight, and this can cause adverse reactions in some patients. In view of these limitations, defined replacements for IVIG are needed.

Although the mechanisms of action for IVIG remain unclear, Fc-fragments derived from IVIG can cure children suffering from idiopathic thrombotic purpura (ITP) (Debré M *et al,* 1993). Interactions between the Fc portion of IgG with both inhibitory and activating FcyRs found on monocytes and macrophages are considered to be important, although the exact receptors involved are hotly debated (Samuelsson A. et al, 2001; Bazin R. *et al,* 2006; Crow. A.R. *et al,* 2003; Leontyev D. *et al,* 2012; Siragam. V. *et al,* 2006) and may vary for the disease for which IVIG is used (Araujo L.M. *et al,* 2011; Anthony R.M. *et al,* 2011). The inhibitory Fc receptor FcγRIIb has been shown to be necessary for the protective effect of IVIG in a mouse model of ITP (Samuelsson *et al,* 2001). A role of the activating Fc receptor FcγRIIIa has also been demonstrated in various diseases (reviewed Mekhaiel *et al,* 2011b). The FcRn responsible for maintaining the long-half-life of the Fc in plasma has also been postulated to be involved (Roopenian & Akilesh, 2007), although recent work has shown that it is not involved in ameliorating ITP in the mouse model (Crow *et al,* 2011). Fc portions interact not only with Fc receptors, but also certain lectins. It is known that IVIG binds to CD22 lectin (Siglec-2) on B lymphocytes via a terminal sialic acid on the Fc-glycan (Séïté J.F. *et al,* 2010), and a recent study demonstrated that sialylated Fcs are responsible for the anti-inflammatory effects of IVIG in a mouse model of arthritis as a result of their interaction with human lectin receptor, DC-SIGN, on DCs (Anthony R.M *et al,* 2011).

IVIG may also work via a multi-step model where the injected IVIg first forms a type of immune-complex in the patient (Clynes et al, 2005; Siragam et al, 2005, 2006; Machino et al, 2012). Once these immune-complexes are formed, they interact with these receptors to mediate anti-inflammatory effects helping to reduce the severity of autoimmune disease or the inflammatory state (Siragam et al, 2006). Multimers of Fc are formed in IVIG by anti-idiotype interactions (Machino et al, 2010; Machino et al, 2012; Roux & Tankersley, 1990; Teeling et al, 2001) or by covalent interactions of the Fc (Yoo et al, 2003). IgG multimers are postulated to show higher avidity binding to the above receptors and by nature of cross-linking the said receptors induce protective signals that are not induced by monomers of Fc. This is supported by the reversal of ITP in mice by immune-complexes (ICs) (Bazin et al, 2006), and by the observation that ICs enhance tolerogenicity of immature dendritic cells via FcγRIIb to promote attenuation of lupus (Zhang et al, 2011). The proportion of multimeric IgG and/or sialylated IgG in commercially available IVIG is extremely low (< 1% and 5% respectively), which may contribute to the need to administer large quantities (Nimmerjahn & Ravetch, 2007).

Other proposed mechanisms of action of IVIG are restoration of the idiotypic-anti-idiotypic network; suppression or neutralization of cytokines by specific antibodies in the IVIG; blockage of binding of adhesion molecules on leukocytes to vascular endothelium; Inhibition of complement uptake on target tissues; neutralization of microbial toxins; blockage of Fas ligand-mediated apoptosis by anti-Fas antibodies in the IVIG; induction of apoptosis with anti-Fas antibodies at high concentrations of IVIG; neutrophil apoptosis by anti-Siglec-9 antibodies in IVIG; saturation of the FcRn receptors to enhance the clearance of autoantibodies; induction of inhibitory FcγRIIb receptors on effector macrophages; neutralization of growth factors for B cells, such as B-cell activating factor; inhibition of T cell-proliferative responses; expansion, activation, or both of a population of Treg cells; inhibition of the differentiation and maturation of dendritic cells; enhancement of the differentiation and maturation of "primed" dendritic cells (reviewed in Ballow, 2011; Mekhaiel et al, 2011b).

Recombinant proteins for use in treating autoimmune diseases and/or as IVIG replacement compounds have been proposed. US 2011/0081345 (Moore) discloses single chain Fc (scFc) proteins, having one Fc unit per molecule, composed from two linked Fc domain amino acid chains, which may be useful for treating autoimmune disease. US 2004/0062763 (Temple University; Mosser) discloses use of multivalent Abs or portions thereof to ligate FcγRI to upregulate IL-10 production, to treat autoimmune disorders. The agent may be two or more Fc fragments coupled together or provided in a single recombinant peptide. US 2008/0206246 (The Rockefeller University; Ravetch) discloses a polypeptide containing at least one IgG Fc region, which is sialylated, and its use as IVIG replacement compound. Although the compounds disclosed in these documents might target Fc receptors, monomeric or dimeric Fc-containing compounds may not bind to Fc receptors with sufficient avidity to be effective or fully effective as IVIG replacement compounds. They would therefore not be suitable biomimetics of the multimeric fraction of IVIG. Higher order multimers are not disclosed in these documents. Neither is any means of engineering higher order multimers which are active as IVIG replacement compounds.

US 2010/0239633 (also published as WO 2008/151088, University of Maryland, Baltimore; Strome) discloses IVIG replacement compounds comprising multiple linked Fc portions. Star-shaped arrangements are envisaged using the Cµ4 domain of IgM and the J chain to effect polymerisation. However, working examples were not described, and computer simulations reported herein suggest that exemplary molecules would not polymerise effectively and/or that the Fc portions would not be arranged for effective binding of Fc or other receptors. In addition, complex biologicals containing more than one different polypeptide chain may be more difficult to manufacture to uniformity, because not all of the polypeptide subunits may interact in a stable and predictable way. For example, cell lines expressing antibody heavy and light chains which produce active intact antibody containing the correct proportion of light and heavy chains may also produce therapeutically inactive heavy chain dimers without light chain attachment, or halfmer molecules.

Linear structures were also proposed in US 2010/0239633, in which individual amino acid chains dimerize by pairing between identical Fc domain amino acid chains, thus generating Fc regions. For example, hinge regions may form inter-chain disulphide bonds between two individual amino acid chains. However, where multiple Fc amino acid chains are linked in series, there may be multiple different ways in which these chains may pair, as illustrated in Figures 11 and 12 of US 2010/0239633. Thus, a uniform product with reliable and predictable properties would not be expected. It is proposed to include a terminal heavy chain domain from IgE to prevent this "zippering" effect. However, binding of the molecules to IgE receptors could have unwanted consequences, including the risk of anaphylactic shock or other allergic responses.

US 2010/0239633 also suggests making cluster molecules in which multimerizing regions, such as IgG2a hinge or isoleucine zippers, cause amino acid chains to dimerize or multimerize, thus bringing together pairs of Fc domain amino acid chains to form functional Fc units. In a follow on study, Jain et al (2012) describe the production of recombinant proteins in which the human IgG2 hinge sequence or the isoleucine zipper was fused to murine IgG2a. The authors report that either multimerizing region caused the formation of homodimers (i.e. single functional Fc units referred to herein as monomers) and multimers. Multimeric fractions of these proteins had some efficacy in treatment of a mouse model of ITP or in the collagen-induced arthritis mouse model. However, the bulk of the produced protein containing the IgG2 hinge was monomeric, or existed as dimers or trimers. Higher order oligomers accounted for only a small proportion, with unknown quaternary structure. In proteins containing the isoleucine zipper, the proportion of higher order multimers was lower. The higher order multimers appeared heterogeneous in size, and their structures, including the nature of the glycans attached at N297, are unknown. Isolating some or all of these multimers for therapeutic use would necessitate substantial waste given their low proportion in the produced protein, and appears unlikely to be commercially viable.

WO 2012/016073 (Block, Gliknik) also discloses multimerised forms of immunoglobulin Fc.

A need remains for compounds of defined structure which effectively target appropriate mechanisms underlying the biological activity of IVIG, for treating autoimmune and inflammatory diseases.

The listing or discussion of a prior-published document in this specification should not be taken as an acknowledgement that the document is part of the state of the art or is common general knowledge.

### SUMMARY OF THE INVENTION

The invention provides a polymeric protein consisting of six polypeptide monomer units for use in a method of treating an autoimmune or inflammatory disease in a mammalian subject, wherein the method comprises administering to the mammalian subject an effective amount of the polymeric protein;
wherein each polypeptide monomer unit consists of an Fc receptor binding portion consisting of two immunoglobulin G heavy chain constant regions;
wherein each immunoglobulin G heavy chain constant region comprises a cysteine residue which is linked via a disulfide bond to a cysteine residue of an immunoglobulin G heavy chain constant region of an adjacent polypeptide monomer unit; and
wherein a tailpiece region is fused to each of the two immunoglobulin G heavy chain constant regions, wherein the tailpiece regions facilitate the assembly of the monomer units into a polymer.

### BRIEF DESCRIPTION OF THE DRAWINGS

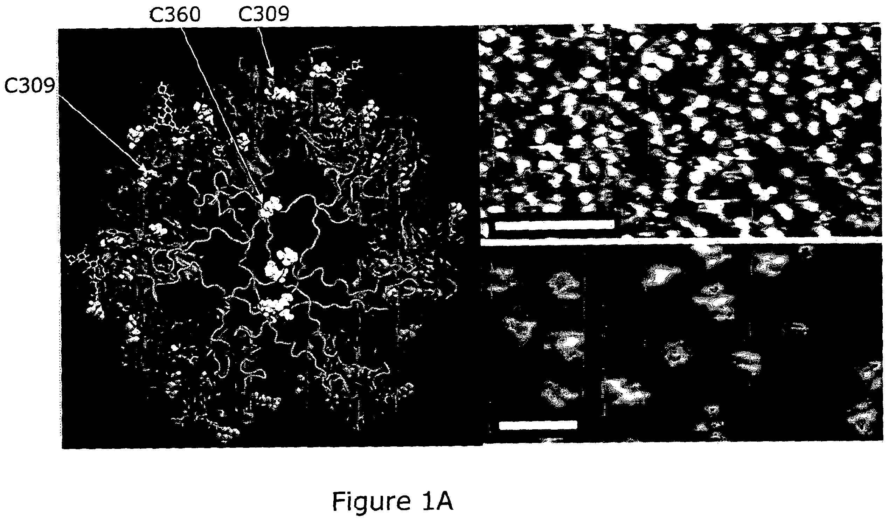

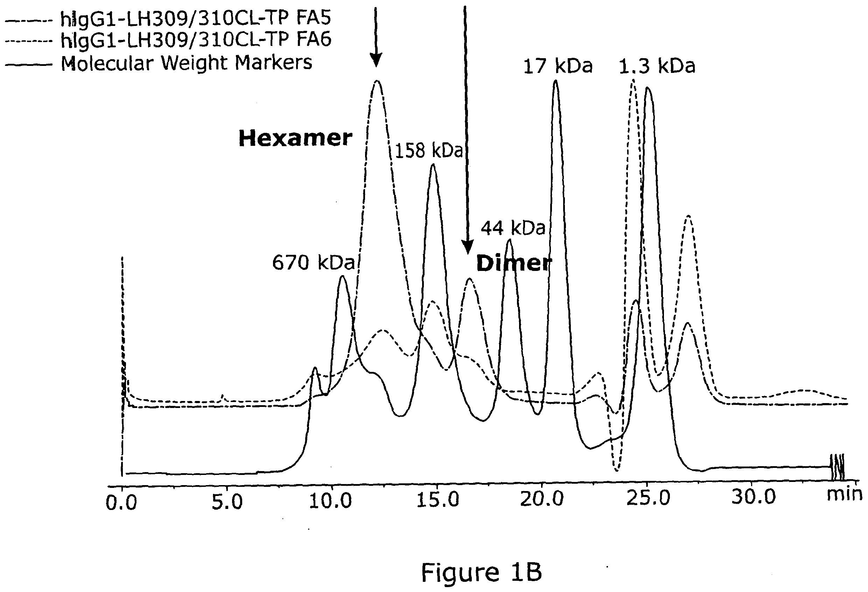

**Fig 1****: Hexameric-Fc and structural characterization. A** Model of hexameric Fc showing Cys309 and Cys360 (tailpiece) disulphide linkages formed between one monomer and its adjacent monomers (left panel). Tapping mode atomic force microscopy images (right hand panels) reveal barrel shaped, six-fold symmetric complexes consistent with hexamers. At smaller scan sizes (lower of the two right hand panels), these complexes are shown to be ∼20nm in diameter (scale bar in lower panel is 50nm). **B** The hexameric-Fc produced from mammalian cell lines can be detected as molecules of approximately 312 kDa by size-exclusion chromatography (trace highlighted as hexamer). A small proportion of dimers is also detected.

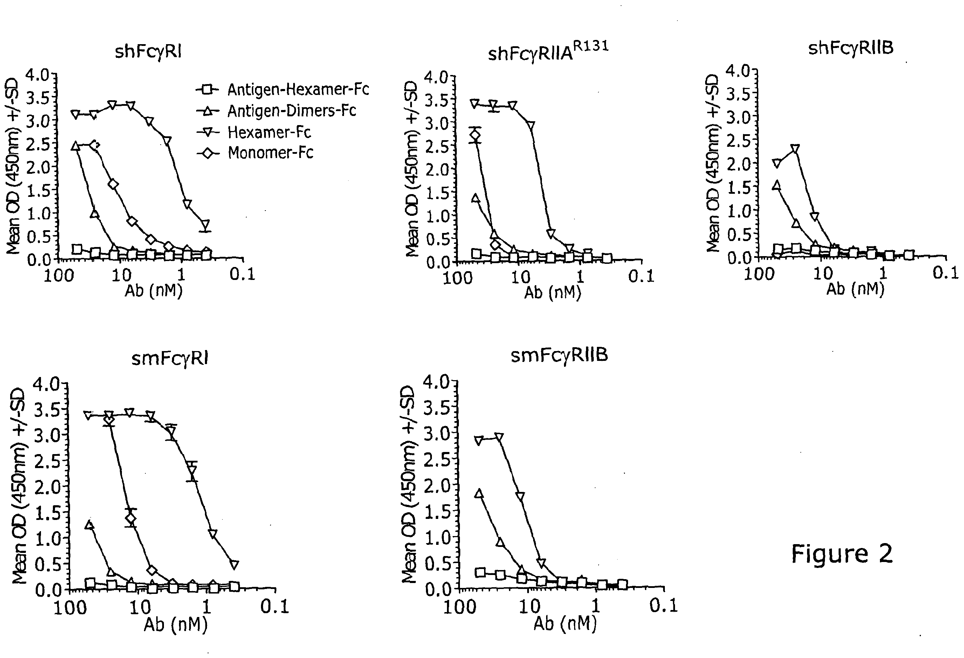

**Fig 2****: Binding of hexameric-Fc to human and mouse Fcγ-receptors (FcyRs).** Titrated amounts (50-0.3 nM of Fc proteins were coated onto ELISA wells. Binding by human or mouse glutathione-S-transferase (GST)-fused FcyRs as indicated were visualized using horse-radish peroxidase (HRP)-conjugated anti-GST antibodies. Values represent triplicate determinations. Error bars are standard errors (SE) around the mean. Antigen-fused Fc proteins bound poorly to Fc receptors, as described in Mekhaiel *et al,* 2011a.

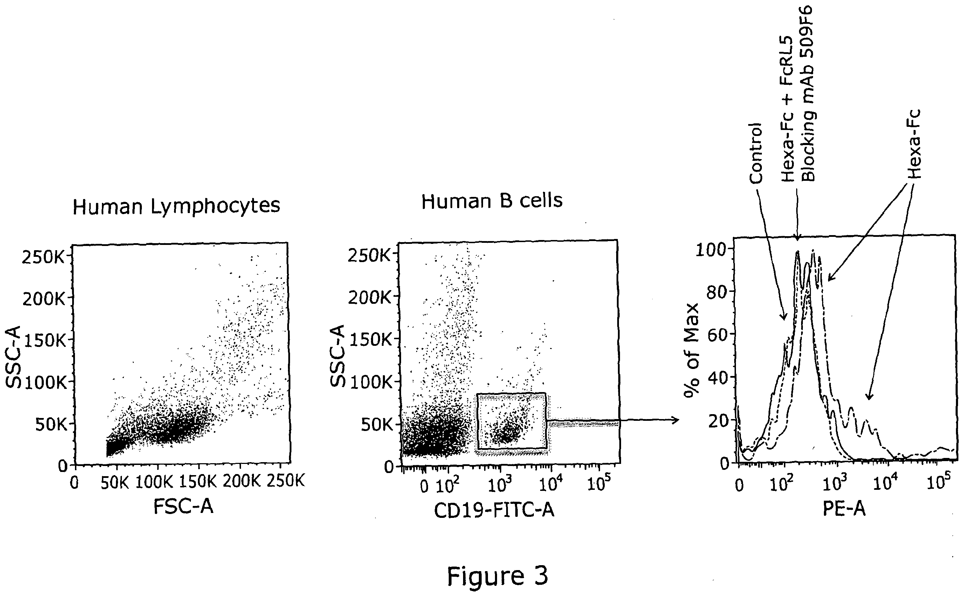

**Fig 3****: Binding of hexameric-Fc to human CD19+ human B lymphocytes.** Characteristic flow cytometry plot showing different populations of human leucocytes represented by their forward and side-scatter profiles (left panel). Individual CD19+ B lymphocytes stained with anti-human CD19-FITC (boxed, middle panel) were gated and investigated for binding of hexameric-Fc (right panel). Binding of 50 µg of hexa-Fc to CD19+ B cells is indicated by the furthest trace on the right (arrowed hexa-Fc). Prior incubation of cells with monoclonal antibody 509F6 specific for FcRL5 (arrowed hexa-Fc + FcRL5 blocking mAb 509F6) ablated binding of hexameric-Fc indicating that FcRL5 was responsible for some of the binding of hexameric-Fc to B cells.

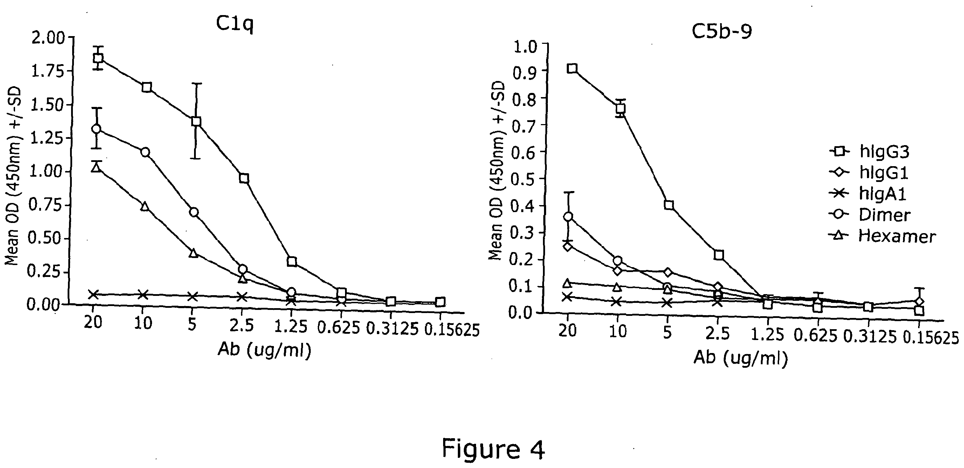

**Fig 4****: Complement binding analysis to Fc proteins.** C1q (left panel) and C5-9 deposition (right panel) to Fc-fusions determined by ELISA. Each point represents the mean optical density (+/-SD) of duplicate wells for each mouse within a given group. Data from one of three replicate experiments are shown.

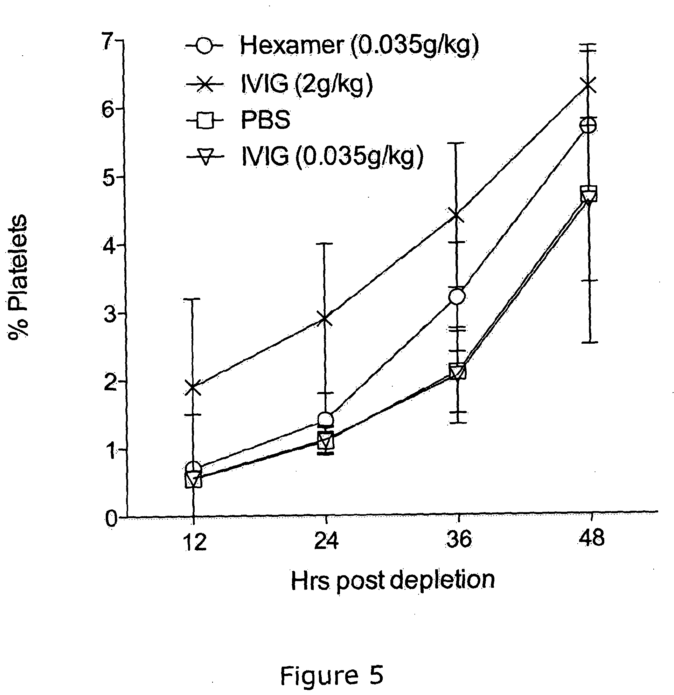

**Fig 5****: Hexameric Fc protein protects against platelet loss in a mouse model of ITP.** Balb/C mice were injected *i.p.* with Hexa-Fc, IVIG (GammaGard), or PBS. One hour later ITP was induced in all mice. Platelets were enumerated at indicated time-points post treatment.

### DETAILED DESCRIPTION OF PREFERRED EMBODIMENTS OF THE INVENTION

The term "immunoglobulin G heavy chain constant region" means a native immunoglobulin G heavy chain region, or variant or fragment thereof. The Fc receptor binding portion typically comprises the Fc portion of an immunoglobulin G, or fragment or variant thereof. The term "Fc portion" includes a fragment of an IgG molecule which is obtained by limited proteolysis with the enzyme papain, which acts on the hinge region of IgG. An Fc portion obtained in this way contains two identical disulphide linked peptides containing the heavy chain CH2 and CH3 domains of IgG, also referred to as Cγ2 and Cγ3 domains respectively. The two peptides are linked by two disulphide bonds between cysteine residues in the N-terminal parts of the peptides. The arrangement of the disulphide linkages described for IgG pertain to natural human antibodies. There may be some variation among antibodies from other mammalian species, although such antibodies may be suitable in the context of the present invention. Antibodies are also found in birds, reptiles and amphibians, and they may likewise be suitable. Nucleotide and amino acid sequences of human Fc IgG are disclosed, for example, in Ellison et al. (1982) NUCLEIC ACIDS RES. 10: 4071-4079. Nucleotide and amino acid sequences of murine Fc IgG2a are disclosed, for example, in Bourgois et al. (1974) EUR. J. BIOCHEM. 43: 423-435. Immunoglobulin G heavy chain constant regions may typically be produced by recombinant expression techniques, and associate as monomer units by disulphide linkages, as occurs in native antibodies. Alternatively, the two constant regions may be produced as a single amino acid chain with an intervening linker region, i.e. as a single chain Fc (scFc) typically also by recombinant expression techniques. scFc molecules are described in US 2011/0081345, including examples having the following general structure from N to C terminus: Hinge-CH2-CH3-linker-Hinge-CH2-CH3.

Typically, each of the immunoglobulin G heavy chain constant regions comprises an amino acid sequence of a mammalian heavy chain constant region, preferably a human heavy chain constant region; or variant thereof. A suitable human IgG subtype is IgG1.

The Fc receptor binding portion may comprise more than the Fc portion of an immunoglobulin. For example, it may include the hinge region of the immunoglobulin which occurs between CH1 and CH2 domains in a native immunoglobulin. For certain immunoglobulins, the hinge region is necessary for binding to Fc receptors. Preferably, the Fc receptor binding portion lacks a CH1 domain and heavy chain variable region domain (VH). The Fc receptor binding portion may be truncated at the C- and/or N-terminus compared to the Fc portion of the corresponding immunoglobulin. Such a Fc receptor binding portion is thus a "fragment" of the Fc portion.

The polymeric protein is formed by virtue of each immunoglobulin G heavy chain constant region comprising a cysteine residue which is linked via a disulfide bond to a cysteine residue of an immunoglobulin G heavy chain constant region of an adjacent polypeptide monomer unit. Because IgM and IgA are naturally polymeric, whereas IgG is naturally monomeric, the ability of monomer units based on IgG heavy chain constant regions to form polymers may be improved by modifying the parts of the IgG heavy chain constant regions to be more like the corresponding parts of IgM or IgA. Suitably, each of the immunoglobulin heavy chain constant regions or variants thereof is an IgG heavy chain constant region comprising an amino acid sequence which comprises a cysteine residue at position 309 according to the EU numbering system, and preferably also a leucine residue at position 310. The EU numbering system for IgG is described in Kabat EA et al, 1983 Sequences of proteins of immunological interest. US Department of Health and Human Services, National Institutes of Health, Washington DC. Sørensen et al (1996) J Immunol 156: 2858-2865 describes the mutation of Leu 309 to Cys 309 in a human IgG3 molecule comprising a IgM tailpiece, to promote polymer formation. Leu 309 corresponds by sequence homology to Cys 414 in Cµ3 domain of IgM and Cys 309 in the Cα2 domain of IgA. (Note that the numbering of amino acid residues differs between the IgM and IgA or IgG classes.) Other mutations may also be advantageous.

Each polypeptide monomer unit comprises a tailpiece region fused to each of the two immunoglobulin G heavy chain constant regions; wherein the tailpiece region of each polypeptide monomer unit facilitates the assembly of the monomer units into a polymer. Typically, the tailpiece region is fused C-terminal to each of the two immunoglobulin heavy chain constant regions. Suitably, the tailpiece region is an IgM or IgA tailpiece, or fragment or variant thereof. These tailpieces are the final portions of the Cµ4 domain of IgM or the Cα3 domain of IgA respectively.

Where a region is described as being fused C-terminal to another region, the former region may be fused directly to the C-terminus of the latter region, or it may be fused to an intervening amino acid sequence which is itself fused to the C-terminus of the latter region. N-terminal fusion may be understood analogously.

An intervening amino acid sequence may be provided between the heavy chain constant region and the tailpiece, or the tailpiece may be fused directly to the C-terminus of the heavy chain constant region. For example, a short linker sequence may be provided between the tailpiece region and immunoglobulin heavy chain constant region. Typical linker sequences are of between 1 and 20 amino acids in length, typically 2, 3, 4, 5, 6 or up to 8, 10, 12, or 16 amino acids in length. A suitable linker to include between the heavy chain region and tailpiece region encodes for Leu-Val-Leu-Gly.

A preferred tailpiece region is the tailpiece region of human IgM, which is PTLYNVSLVMSDTAGTCY (Rabbitts TH et al, 1981. Nucleic Acids Res. 9 (18), 4509-4524; Smith et al (1995) J Immunol 154: 2226-2236). Suitably, this tailpiece may be modified at the N-terminus by substituting Pro for the initial Thr, thus generating the sequence PPLYNVSLVMSDTAGTCY. This does not affect the ability of the tailpiece to promote polymerisation of the monomer. Further suitable variants of the human IgM tailpiece are described in Sørensen et al (1996) J Immunol 156: 2858-2865. A further IgM tailpiece sequence is GKFTLYNVSLIMSDTGGTCY from rodents (Abbas and Lichtman, Cellular and Molecular Immunology, Elsevier Saunders, 5th Edn, 2005). An alternative preferred tailpiece region is the tailpiece region of human IgA, which is PTHVNVSVVMAQVDGTCY (Putnam FW et al, 1979, J. Biol. Chem 254: 2865-2874)] Other suitable tailpieces from IgM or IgA of other species, or even synthetic sequences which facilitate assembly of the monomer units into a polymer, may be used. It is not necessary to use an immunoglobulin tailpiece from the same species from which the immunoglobulin heavy chain constant regions are derived, although it is preferred to do so.

"Variants" and "fragments" are as defined in relation to heavy chain constant regions. A variant of an IgM tailpiece typically has an amino acid sequence which is identical to PPLYNVSLVMSDTAGTCY in 8, 9, 10, 11, 12, 13, 14 ,15, 16 or 17 of the 18 amino acid positions. A variant of an IgA tailpiece typically has an amino acid sequence which is identical to PTHVNVSVVMAQVDGTCY in 8, 9, 10, 11, 12, 13, 14 ,15, 16 or 17 of the 18 amino acid positions. Fragments of these IgM or IgA tailpieces typically comprise 8, 9, 10, 11, 12, 13, 14 ,15, 16 or 17 amino acids. Fragments of variants are also envisaged. Typically, fragments and variants of the IgM or IgA tailpiece retain the penultimate cysteine residue, as this is believed to form a disulphide bond between two monomer units in a polymeric protein.

The ability of a given tailpiece region to facilitate assembly of the monomer units into a polymer may be tested by comparing the proportion of protein having a high molecular size when formed from monomer units lacking a tailpiece with monomer units comprising a tailpiece. The latter may form a higher proportion of high molecule size polymers under native conditions. Native molecular weights can be determined by size-exclusion chromatography, for example on Sephadex-200 columns on an AKTA FPLC (Amersham). Alternatively, non-reducing gel electrophoresis may be used, as described in Smith *et al (supra) or* Sørensen *et al (supra).*

When the monomer units have assembled into a polymer, the Fc receptor binding portions are arranged in a planar polymeric structure which is spatially orientated to allow each Fc receptor binding portion to bind to an Fc receptor. IgM is naturally pentameric or hexameric and IgA naturally forms dimers, trimers or tetramers. These properties appear to be determined, at least in part, by the ability of the tailpiece to cause the monomers to associate into polymers. Pentameric IgM is formed when the IgM associates with the J chain, although it is typically hexameric in the absence of the J chain. The J chain may or may not be included as a further component of the polymeric fusion protein of the invention. Production is simplified by omitting the J chain polypeptide, which in any case, is not needed for polymerisation of IgG (Ghumra et al, 2008). Secretory IgM or IgA found at mucosal surfaces contains secretory component (SC), part of the polymeric Ig-receptor used to translocate them from blood to secretions. The SC may or may not be included as a further component of the polymeric protein of the invention. Production is simplified by omitting the SC which, in any case, is not needed for polymerisation.

The polymeric protein comprising six polypeptide monomer units is used according to the first aspect of the invention. The exemplary Fc protein comprising human IgG1 heavy chain constant regions and a human IgM tailpiece described herein, forms hexamers and dimers. Variants based on other IgG heavy chain constant regions, including different tailpieces or no tailpiece, may form polymers having different numbers of monomers. In particular, they may be pentamers or heptamers rather than hexamers. Where Fc proteins naturally associate into polymers having different numbers of monomer units, polymers having the required number of monomer units can be separated according to molecular size, for example by gel filtration. Mixtures where at least a proportion of the protein is in the form of a hexamer may also be used. Suitably hexamers are used in the absence of proteins having other numbers of monomer units.

In the polymeric proteins to be used according to the first aspect of the invention, the Fc receptor binding portion of each monomer unit is capable of binding to an Fc receptor. It will be appreciated that Fc receptor binding portions which comprise the Fc portion of a particular immunoglobulin, will bind to different Fc receptors depending on the binding specificity of the particular immunoglobulin. Typically, the Fc receptor binding portion will have an affinity for a given Fc receptor which is at least comparable to the affinity of a native monomeric immunoglobulin molecule. However, lower affinities may be tolerated because the polymeric protein comprises multiple such Fc receptor binding portions, and will therefore bind to Fc receptors with higher avidity. Therefore, the Fc receptor binding portion will typically have an affinity which is at least a tenth, suitably at least a fifth and most suitably at least a half of the affinity of the corresponding native monomeric immunoglobulin molecule which binds to the given Fc receptor.

Affinity constants can be readily determined by surface Plasmon Resonance Analysis (Biacore). The Fc receptor binding portions can be passed over flow cells from CM5 sensor chips amine-coupled to Fc receptors. Equimolar concentrations of the Fc receptor binding portion or intact monomeric antibody may be injected over each Fc receptor and association and dissociation observed in real time. Data from a BIAcore X or 3000 machines may be analyzed using BIAevaluation 3.0 software to determine accurate affinity constants.

There are three classes of human Fcγ receptor (Gessner et al (1998) Ann Hematol 76: 231-48; Raghavan and Bjorkman (1996) Ann Rev Cell Dev Biol 12: 181-220). FcγRI (CD64) binds monomeric IgG with high affinity. FcγRII (CD32) and FcγRIII (CD16) are the low affinity receptors for Fc and can only interact with high affinity with antibodies that are presented to the immune system as immune complexes (ICs). Although larger (>350 kDa) multimeric IgG and circulating ICs are mostly removed in the preparation of IVIG, these are common in healthy individuals where they can contribute as much as 10% to the overall plasma Ab concentration, indicating a physiological role in maintaining immune homeostasis in these healthy individuals (Nezlin R (2009) Immunol Lett 122,141-4).

FcγRII and FcγRIII are closely related in the structure of their ligand-binding domains. In humans three separate genes, FcγRIIA, FcγRIIB, and FcγRIIC, two of which give rise to alternatively spliced variants, code for FcγRII. FcγRIIa delivers activating signals whereas FcγRIIb delivers inhibitory signals. The functional basis for the divergent signals arises from signaling motifs located within the cytoplasmic tails of the receptors. An immunoreceptor tyrosine-based inhibitor motif (ITIM) located in the cytoplasmic tail of the FcγRIIb is involved in negative receptor signaling. The ITIM motif is a unique feature of the FcγRIIb receptor as it is not apparently present in any other Fcγ receptor class. In contrast, an activatory immunoreceptor tyrosine-based activation motif or ITAM is located in the cytoplasmic tail of FcγRIIa. ITAM motifs transduce activating signals. They are also found in the FcRγ-chains, which are identical to the y chains of the high affinity IgE receptor (FcεRI). While FcγRIIa and FcγRIIb are widely expressed on myeloid cells and some T-cell subsets they are notably absent from NK cells. There are two alleles for the FcγRIIa receptor in humans, referred to as His131 (H131) and Arg131 (R131). The FcγRIIa-Arg131 allele is associated with increased susceptibility to infection by encapsulated bacteria, such as *Haemophilus influenzae, Streptococcus pneumoniae* and *Neiserria meningitidis,* which elicit IgG2 responses (Pleass RJ & Woof JM, 2001). The Fc receptor encoded by this allele cannot bind IgG2 and is therefore unable to elicit clearance of IgG2-coated bacteria. Both variants bind human IgG1, however.

Human FcγRIII is also present in multiple isoforms derived from two distinct genes (FcyRIIIA and FcγRIIIB). FcγRIIIb is unique in its attachment to the cell membrane via a glycosylphosphatidyl anchor. FcγRIIIb expression is restricted to neutrophils while FcγRIIIa is expressed by macrophages, and NK cells. FcγRIIIa is also expressed by some T-cell subsets and certain monocytes. FcγRIIIa requires the presence of the FcRγ-chain or the CD3ζ-chain for cell surface expression and signal transduction. The FcRγ-chain and the CD3ζ-chain are dimeric and possess ITAM motifs. FcγRIIIa forms a multimeric complex with these subunits and signalling is transduced through them. Thus, there is considerable FcyR receptor heterogeneity and diverse expression

The binding sites for FcγRII and FcγRIII map to the hinge and proximal region of the CH2 domain of IgG, the same region originally identified for FcγRI (Duncan et al (1988) Nature 332: 563-4; Morgan et al (1995) Immunol 86: 319-324; Lund et al (1991) J Immunol 147: 2657-2662).

Fcγ receptors (FcγRs) trigger activatory and/or inhibitory signalling pathways that set thresholds for cell activation and culminate in a well-balanced immune response (Nimmerjahn F & Ravetch JV (2008) Nat. Rev. Immunol. 8: 34-47). Activating and inhibitory FcRs are widely expressed throughout the haematopoetic system but particularly on professional antigen presenting cells (APCs) (Nimmerjahn F & Ravetch JV (2008) supra). For example in humans, FcγRI is constitutively expressed by blood myeloid dendritic cells (DCs) and FcγRII has been detected on every DC subset examined to date, whereas the expression of FcγRI, FcγRIIB and FcγRIII dominate on murine DCs (Ravetch JV (2003) in Fundamental Immunology (ed. Paul WE) 685-700 (Lippincott-Raven, Philidelphia); Bajtay Z et al (2006) Immunol. Lett. 104: 46-52). FcyRs also play a pre-eminent role in antigen presentation and immune-complex-mediated maturation of dendritic cells (DCs), and in regulation of B-cell activation and plasma-cell survival (Ravetch JV (2003) *supra;* Bajtay Z et al, (2006) *supra*). Moreover, by regulating DC activity, FcyRs control whether an immunogenic or tolerogenic response is initiated after the recognition of antigenic peptides presented on the surface of DCs to cytotoxic T cells, T helper cells, and regulatory T cells. FcγRs also co-operate with Toll-like receptors (TLRs) in controlling levels of the important regulatory cytokines, IL-12 and IL-10 (Polumuri SK, 2007, J. Immunol 179: 236-246). Thus, FcγRs are involved in regulating innate and adaptive immune responses, which makes them attractive targets for the development of novel immunotherapeutic approaches (Nimmerjahn F & Ravetch JV (2008) *supra*).

It is known that the inhibitory FcγRIIB controls the magnitude of the immune response, as DCs derived from FcγRIIB-knockout mice generate stronger and longer-lasting immune responses *in vitro* and *in vivo* (Bergtold A, Desai DD, et al (2005) Immunity 23: 503-514; Kalergis A M & Ravetch JV. (2002) J. Exp. Med. 195: 1653-1659). More importantly, FcyRIIB-deficient DCs or DCs incubated with a mAb that blocks immune complex binding to FcγRIIB showed a spontaneous maturation (Boruchov AM, et al (2005) J. Clin. Invest. 115: 2914-2923; Dhodapkar KM, et al (2005) Proc. Natl Acad. Sci. USA 102: 2910-2915). This suggests that the inhibitory FcγR not only regulates the magnitude of cell activation but also actively prevents spontaneous DC maturation under non-inflammatory steady-state conditions. Indeed, low levels of immune complexes can be seen in the serum of healthy donors, emphasizing the importance of regulatory mechanisms that prevent unwanted DC activation (Dhodapkar KM, et al (2005) Proc. Natl Acad. Sci. USA 102: 2910-2915). The loss of FcγRIIB also results in the priming of more antigen-specific T cells (Kalergis A M & Ravetch JV. (2002) J. Exp. Med. 195: 1653-1659). Therefore the binding of the polymeric Fc protein to multiple copies of FcγRIIB may induce negative responses from cells expressing this receptor.

FcRL5 is a recently described Fc receptor capable of inhibitory signalling, which is expressed on B cells and which binds aggregated IgG but not monomeric IgG (Wilson et al, 2012). These authors propose that IgG may bind cooperatively to FcRL5 and FcγRIIb co-expressed on B cells. As described in Example 3, an exemplary hexameric protein can bind FcRL5 independently of FcγRIIb.

Where the Fc receptor binding portion comprises immunoglobulin heavy chain constant regions of a human IgG isotype or variants thereof, it will typically bind to human Fcγ-receptors (FcγRI, FcγRII and FcγRIII) and/or human FcRL5. Surface Plasmon Resonance Analysis as described above can be used to determine affinity constants. Typical affinity constants for binding of human IgG1 or IgG3 to FcγRI are about 10⁻⁹ M; for FcγRII are about 0.6-2.5x10⁻⁶ M; for FcγRIIIA are about 5x10⁻⁵ M; for FcγRIIIB are about 0.6-2.5x10⁻⁶ M. Monomeric IgG does not noticeably bind to FcRL5, although FcRL5 does bind with high avidity to aggregated IgG, suggesting FcRL5 is a low to medium affinity receptor for monomeric IgG with an affinity constant of about 10⁻⁵-10⁻⁶ M (taken from Wilson et al, 2012). Alternatively, ELISAs may be used essentially as described in Example 3 to obtain a semi-quantitative indication of binding properties.

Fc receptor binding portions also typically bind to lectins, also referred to as glycan receptors, particularly lectins which bind to sialic acid. Exemplary lectins are human DC-SIGN (or mouse homologue SIGN-R1) and CD22, which contribute to the therapeutic properties of IVIG (reviewed in Mekhaiel, 2011b). Polymeric proteins are predicted to interact with these receptors, as explained in Example 3. SIGN-R1 binding can be determined by surface plasmon resonance as described in Jain *et al,* 2012. Analogous methods may be used to determine binding to CD22 or DC-SIGN. Human 2,6 sialylated Fc binds SIGN-R1 and human DC-SIGN with affinity constants 2.7x10⁻⁶ M and 3.6x10⁻⁶ M respectively (Anthony et al, 2008, PNAS 105: 19571-19578). sialylated Fc does not bind appreciably. Multimeric sialoproteins e.g. haemagglutinin are known to bind to sialic acid receptors with affinities in the order of 10⁻⁸M compared to monomers which bind 10⁻²M (Mammen M, Choi SK, Whitesides GM (1998) Angew Chem Int Edit 37: 2755-2794.) Hence, as an increase in avidity can enhance an inherently weak substrate affinity, presenting terminal sialic acid residues on polymeric proteins would likewise be expected to significantly enhance binding to sialic acid receptors.

The appropriate limits for and determination of affinity constants for Fc receptor binding portions which comprise variants of native immunoglobulin heavy chain constant regions, or fragments of Fc portions, is as described above.

A "variant" refers to a protein wherein at one or more positions there have been amino acid insertions, deletions, or substitutions, either conservative or non-conservative.

A "variant" may have modified amino acids. Suitable modifications include acetylation, glycosylation, hydroxylation, methylation, nucleotidylation, phosphorylation, ADP-ribosylation, and other modifications known in the art. Such modifications may occur postranslationally where the peptide is made by recombinant techniques. Otherwise, modifications may be made to synthetic peptides using techniques known in the art. Modifications may be included prior to incorporation of an amino acid into a peptide. Carboxylic acid groups may be esterified or may be converted to an amide, an amino group may be alkylated, for example methylated. A variant may also be modified post-translationally, for example to remove carbohydrate side-chains or individual sugar moieties e.g. sialic acid groups or to add sialic acid groups.

By "conservative substitutions" is intended combinations such as Val, Ile, Leu, Ala, Met; Asp, Glu; Asn, Gln; Ser, Thr, Gly, Ala; Lys, Arg, His; and Phe, Tyr, Trp. Preferred conservative substitutions include Gly, Ala; Val, Ile, Leu; Asp, Glu; Asn, Gln; Ser, Thr; Lys, Arg; and Phe, Tyr.

Typical variants of the immunoglobulin G heavy chain constant regions will have an amino acid sequence which is at least 70%, typically at least 80%, at least 90%, at least 95%, at least 99% or at least 99.5% identical to the corresponding immunoglobulin G heavy chain constant region of a native immunoglobulin. Suitably, the variant is a variant of the human immunoglobulin G1 heavy chain constant region and has an amino acid sequence which is at least 90%, at least 95%, at least 99% or at least 99.5% identical to the latter.

A "fragment" refers to a protein wherein at one or more positions there have been deletions. Typically a fragment of a Fc portion comprises at least 60%, more typically at least 70%, 80%, 90%, 95% or up to 99% of the complete sequence of the Fc portion. Fragments of variants are also encompassed.

The percent sequence identity between two polypeptides may be determined using suitable computer programs, for example the GAP program of the University of Wisconsin Genetic Computing Group and it will be appreciated that percent identity is calculated in relation to polypeptides whose sequence has been aligned optimally.

The alignment may alternatively be carried out using the Clustal W program (Thompson et al., (1994) Nucleic Acids Res., 22(22), 4673-80). The parameters used may be as follows:
- Fast pairwise alignment parameters: K-tuple(word) size; 1, window size; 5, gap penalty; 3, number of top diagonals; 5. Scoring method: x percent.
- Multiple alignment parameters: gap open penalty; 10, gap extension penalty; 0.05.
- Scoring matrix: BLOSUM.

Variants may be natural or made using the methods of protein engineering and site-directed mutagenesis as are well known in the art.

"Peptides" generally contain up to 10, 20, 50 or 100 amino acids. Peptides and polypeptides may conveniently be blocked at the N- or C-terminus so as to help reduce susceptibility to exoproteolytic digestion. Peptides and polypeptides may be produced by recombinant protein expression or *in vitro* translation systems (Sambrook et al, "Molecular cloning: A laboratory manual", 2001, 3rd edition, Cold Spring Harbor Laboratory Press, Cold Spring Harbor, NY). Peptides may be synthesised by the Fmoc-polyamide mode of solid-phase peptide synthesis as disclosed by Lu et al (1981) J. Org. Chem. 46, 3433 and references therein.

Suitably, in the Fc protein described herein, each of the immunoglobulin G heavy chain constant regions comprises an amino acid sequence which is modified compared to the amino acid sequence of a native immunoglobulin G heavy chain constant region, to modify the affinity of the Fc receptor binding portion for at least one Fc receptor. Typically the affinity of the Fc receptor binding portion for a low affinity inhibitory and/or activatory Fcγ receptor may be increased.

The interactions between IgG and Fc receptors have been analyzed in biochemical and structural studies using wild type and mutated Fc. One consensus indicates that some regions for binding to Fc receptors are located in the part of the hinge region closest to the CH2 domain and in the amino-terminus of the CH2 domain that is adjacent to the hinge, including for example residues 233-239 (Glu-Leu-Leu-Gly-Gly-Pro-Ser). Mutations within this region can result in altered binding to Fc receptors. This region appears to be responsible for some of the direct interactions with Fc receptors (Woof JM & Burton D, Nature Reviews Immunology 2004, 4: 89-99). Further into the CH2 domain, and away from the hinge, are other residues that may, at least in some contexts, contribute to Fc receptor binding, including for example, Pro-329 of human IgG1 (EU numbering) which appears to be involved in direct contact with the Fc receptor and Asn-297 which appears to be the sole site for N-linked glycosylation within the Fc region of human IgG1. The presence of carbohydrate at this residue may contribute to the binding to Fc receptors.

Activatory Fcγ receptors are as described above; in the human, the low affinity activatory Fcγ receptors are FcγRIIA/C and FcyRIIIA. FcγRI is the high affinity activatory receptor. FcγRIIB is an inhibitory human Fc receptor. As the ligand binding properties of FcγRIIB and FcγRIIA/C are the same, it may not be possible to increase affinity for FcγRIIA/C whilst simultaneously decreasing affinity for FcγRIIB. Lazar et al (2006) PNAS 103: 4005-10 describes mutations in the Fc portion of a human IgG which affect binding affinity to different Fc receptors. A wildtype IgG bound to FcγRIIIa with a K_{D} of 252 nM; the K_{D} of a I332E mutant was 30 nM and the K_{D} of a S239D/I332E mutant was 2 nM. Combination of an A330L mutation with S239D/I332E increased FcγRIIIa affinity and reduced FcγRIIb affinity. Shields RL et al (2001) J. Biol. Chem. 276: 6591-6604 describes mutations in the Fc portion of human IgG1 which affect binding affinity to different Fc receptors. The S298A mutation increased affinity for FcγRIIIa and decreased affinity for FcγRIIA; the E333A mutation increased affinity for FcγRIIIa and decreased affinity for FcγRIIA; the mutation K334A increased affinity for FcγRIIIa. Any or all of the above mutations may be used individually or in combination. Other suitable mutations may be identified by routine methods.

The affinity of the Fc receptor binding portion for CD22 or DC-SIGN/SIGN-R1 may be increased by increasing the amount of bound sialic acid on the Fc receptor binding portion. Typically this is achieved by increasing the amount or proportion of terminal sialic acid residues on the N297 glycan, which may be added post translationally to the Fc peptide in the endoplasmic reticulum.

Other mutations may suitably be made to improve the efficacy of the Fc receptor binding portions. Suitably, each of the immunoglobulin G heavy chain constant regions comprises an amino acid sequence which is modified compared to the amino acid sequence of a native immunoglobulin G heavy chain constant region, to increase the *in vivo* half life of the polymeric protein, suitably by increasing the affinity of the Fc receptor binding portion for neonatal Fc receptor. Increasing the serum persistence allows higher circulating levels, less frequent administration and reduced doses. This can be achieved by enhancing the binding of the Fc region to neonatal FcR (FcRn). FcRn, which is expressed on the surface of endothelial cells, binds the Fc in a pH-dependent manner and protects it from degradation. Although hexameric-Fc described in the Examples was unable to bind human FcRn it did bind very well to mouse FcRn. The amino acid substitutions M252Y/S254T/T256E and/or H433K/N434F may be introduced into the Fc-receptor binding portion to increase *in vivo* half life of IgG without unduly affecting FcyR interactions (Vaccaro C, et al (2005) Nat. Biotech. 23: 1283-1288). In addition or in the alternative, H310 may be reintroduced.

The binding of multiple Fc receptors by a polymeric Fc protein may cause different intracellular signalling phenomena than the binding of a single Fc receptor by a monomeric Fc protein or IgG. Binding of multiple Fc receptors by the polymeric protein may be more effective in blocking the binding of the Fc receptors to other ligands, particularly in the case of low affinity Fc receptors. The efficacy of the polymeric protein can be compared against the efficacy of a monomeric unit which does not form polymers, in many ways. In such tests, it is typical for the monomeric units of the polymeric Fc protein to, individually, have the same affinity for a given Fc receptor as the monomeric unit which does not form polymers.

Polymeric proteins will have greater avidity for low affinity receptors including Fc receptors than will the control monomeric units. They may also have a greater avidity for FcRL, FcRn, CD22, DC-SIGN or other Fc-receptors. Avidity is the overall binding strength of a polyvalent interaction. The interaction between a Fc binding region and a Fc receptor has a characteristic affinity, whereas the avidity of the interaction increases almost geometrically for each interaction. For low affinity Fc receptors, the increase in binding strength may allow a biologically relevant interaction with a polymeric protein, which could not be achieved by a monomeric or dimeric unit. Multivalent binding by polymeric proteins results in a considerable increase in stability as measured by the equilibrium constant (L/mol), compared to binding of a control monomeric protein. For example, a typical monovalent interaction between an Fc portion and an Fc receptor may have an equilibrium constant of about 10⁴ L/mol. A hexavalent interaction may provide for an equilibrium constant of about 10¹¹ L/mol. The equilibrium constant may vary depending on the Fc portion and the Fc receptor. However, a hexameric protein will typically exhibit an increase in the binding energy compared to a control monomeric protein of up to about 10⁴, 10⁵ or 10⁶ fold, or even greater than 10⁶ fold. Similar increases in binding energy and equilibrium constants may be expected for multivalent interactions with the FcRL5, FcRn, CD22 or DC-SIGN. For description of avidity and affinity see textbook Immunology by Roitt, Brostoff and Male, 2^{nd} edition 1989.

The avidity for Fc receptors of the polymeric protein may be compared to that of the monomeric unit which does not form polymers by Surface Plasmon Resonance Analysis (Biacore), as described above.

It has been found that fusion of a bulky antigen to the polymeric protein can diminish the ability of the polymeric protein to bind to Fc receptors (Mekhaiel et al, 2011a). Suitably, the polymeric protein for use according to the invention does not comprise (for example by being fused or conjugated to) a moiety which reduces binding avidity for FcγRII. The effect on avidity can be determined by comparing binding avidity for a given receptor by the polymeric protein comprising the moiety to be tested with the avidity achieved by the polymeric protein lacking the moiety. Typically, if the avidity is reduced by more than 10-fold, or more than 5-fold or more than 2-fold, the polymeric protein comprising the moiety may not be suitable. Reduced binding avidity for FcγRII would also be indicative of reduced binding avidity for other Fc receptors, including FcγRI or FcγRIII. In the alternative, binding avidity for either or both of these other receptors could be tested.

Increased avidity of polymeric proteins for low-affinity receptors, as described in Example 3, may engage the biological mechanisms underlying IVIG therapy, particularly those mediated by low-affinity receptors FcγRIIB, FcγRIIIA, CD22 and DC-SIGN. Increased avidity for the high affinity IgG receptor FcγRI was also observed for the exemplary hexameric protein. FcγRI is implicated in inflammatory autoimmune disease (Hussein OA et al, Immunol Invest 2010, 39:699-712) and FcγRI-directed immunotoxins inhibit arthritis (Van Vuuren AJ et al, J. Immunol 2006 176: 5833-8). Also MicroRNA-127 inhibits lung inflammation by targeting FcγRI (Xie T et al, J. Immunol 2012 188, 2437-44). Moreover, ligation of FcγRI on macrophages has been proposed to induce production of the anti-inflammatory cytokine IL-10 in US 2004/0062763 (Temple Univeristy; Mosser). Thus, ligation of FcγRI may also have a beneficial effect in the context of the present invention, although FcγRI is not thought to underlie the effects of IVIG.

The immunomodulatory properties of the polymeric protein arise from interactions between the Fc receptor binding portions and Fc receptors and/or other receptors and components of the immune system which interact with Fc portions. The polymeric protein for use according to the first aspect of the invention does not comprise a further immunomodulatory portion; or an antigen portion that causes antigen-specific immunosuppression when administered to the mammalian subject. Contrary to therapeutic approaches which provide immunosuppressive agents such as TNF-receptor, the polymeric proteins described herein rely on a different therapeutic mechanism, which may make them more widely useful in therapy. Neither do the polymeric proteins rely on an antigen-specific immunosuppressive effect, which limits the application of other therapeutic approaches to specific diseases.

An "immunomodulatory portion" is an agent which has an immunomodulatory activity in a healthy or diseased mammalian subject when covalently linked to a polymeric protein as described herein, or when present in the absence of said polymeric protein. "Immunomodulatory activity" refers to altering an immune response in a subject, to increase or decrease components of the immune system such as cytokines or antibodies; or to increase or decrease immune functions such as antigen presentation. An "immunomodulatory" portion may be a chemokine or chemokine receptor, cytokine or cytokine receptor, Toll like receptor (TLR), acute phase protein, complement component, immune receptor, CD molecule, or signal transduction molecule, for example. Such agents are known to possess immunomodulatory activities in a healthy or diseased mammalian subject in the absence of the polymeric protein. Examples of such agents are described in Immunology textbooks such as (Abbas and Lichtman, Cellular and Molecular Immunology, Elsevier Saunders, 5th Edn, 2005), and the skilled worker can find further examples in relevant literature. Thus, conjugates or fusion proteins comprising any of these agents and the polymeric protein are excluded from the invention. Immunomodulatory portions found in monomeric Fc fusion proteins known as etanercept, alefacept, abatacept, belatacept, atacicept, briobacept, rilonacept or afilbercept are particularly excluded. Antibody fragments such as Fab or scFvs may also be regarded as "immunomodulatory portions". Such fragments may bind to components of the immune system, thus having an immunomodulatory activity. When fused or conjugated to monomeric units of the polymer protein, antibody fragments may recapitulate the complete antibody structure i.e. Fc and antigen-combining regions. Such agents may have a more pronounced immunomodulatory activity when covalently linked to the polymeric protein, than when present in isolation. Immunomodulatory portions found in therapeutic antibodies, such as the antibody known as Remicade which binds TNFα, are particularly excluded. Suitably, the polymeric protein does not comprise any antibody portions other than the immunoglobulin G heavy chain constant regions and optionally hinge region, whether immunomodulatory or not.

As noted above, the immunomodulatory properties of the polymeric protein arise from interactions between the Fc receptor binding portions and Fc receptors and/or other receptors and components of the immune system which interact with Fc portions. Modifications of the Fc receptor binding portions, such as modified glycosylation, which modify binding to Fc receptors, lectins or other immune system components are not regarded as further immunomodulatory portions, but as components of the Fc receptor binding portions. Therefore, they are not excluded from the invention.

A suitable experimental test for an immunomodulatory portion is to provide a polymeric protein lacking the putative immunomodulatory portion and a polymeric protein comprising the putative immunomodulatory portion and test either protein for efficacy in the mouse model of ITP described in Example 4. If the putative immunomodulatory portion possesses an immunomodulatory activity, it may alter parameters of the response. For example, it may alter the rate or extent of platelet recovery.

An "antigen" is a molecule that binds specifically to an antibody or a TCR. Antigens that bind to antibodies include all classes of molecules, and are called B cell antigens. Exemplary types of molecule include peptides, polypeptides, glycoproteins, polysaccharides, gangliosides, lipids, phospholipids, DNA, RNA, fragments thereof, portions thereof and combinations thereof. TCRs bind only peptide fragments of proteins complexed with MHC molecules; both the peptide ligand and the native protein from which it is derived are called T cell antigens. "Epitope" refers to an antigenic determinant of a B cell or T cell antigen. Where a B cell epitope is a peptide or polypeptide, it typically comprises three or more amino acids, generally at least 5 and more usually at least 8 to 10 amino acids. The amino acids may be adjacent amino acid residues in the primary structure of the polypeptide, or may become spatially juxtaposed in the folded protein. T cell epitopes may bind to MHC Class I or MHC Class II molecules. Typically MHC Class I-binding T cell epitopes are 8 to 11 amino acids long. Class II molecules bind peptides that may be 10 to 30 residues long or longer, the optimal length being 12 to 16 residues. Peptides that bind to a particular allelic form of an MHC molecule contain amino acid residues that allow complementary interactions between the peptide and the allelic MHC molecule. The ability of a putative T cell epitope to bind to an MHC molecule can be predicted and confirmed experimentally (Dimitrov I et al, Bioinformatics. 2010 Aug 15;26(16):2066-8).

The polymeric protein for use according to the first aspect of the invention does not comprise an antigen portion that causes antigen-specific immunosuppression when administered to the mammalian subject. By "comprise", we include antigens which are covalently linked to at least one of the peptide monomer units, such as by chemical conjugation or recombinant fusion. Typically, the polymeric protein does not comprise a portion that acts as an antigen, whether by causing immunosuppression or immunostimulation, when administered to the mammalian subject.

By "antigen-specific immunosuppression", we include suppression of antibody responses and/or suppression of T cell responses. T cell responses may be suppressed by clonal deletion of antigen-reactive T cells, anergy or suppression, such as via induction of regulatory T cells. T cell responses may also be deviated from more aggressive to less aggressive forms, for example from Th1 type responses to Th2 type responses.

A suitable test for an antigen portion that causes antigen-specific immunosuppression is to provide a polymeric protein lacking the antigen portion and a polymeric protein comprising the antigen portion and testing either protein in the mammalian subject to be treated for the autoimmune or inflammatory disease. Polymeric proteins lacking or containing the antigen may be administered sequentially in the same mammalian subject, and the subject monitored for an immune response to the antigen following treatment with either protein. Alternatively, groups of like subjects may be treated either with the polymeric protein lacking the antigen or the polymeric protein comprising the antigen. Conventional techniques may be used to identify and monitor the immune response to the antigen, whether a B cell response or a T cell response. If there is no difference in the extent or type of immune response to the antigen in the subjects following administration of the polymeric protein lacking the antigen, compared to the polymeric protein comprising the antigen, then the antigen does not cause antigen-specific immunosuppression when administered to the mammalian subject. Thus, antigen-specific means of immunosuppression resulting from an antigen portion of a polymeric protein are excluded. That does not mean that the immune response to a particular antigen may not decrease in response to therapy. The immune response to an auto-antigen implicated in an autoimmune disease may decrease in response to therapy, but this can be achieved by the polymeric protein lacking the antigen equally as well as by a polymeric protein comprising the antigen. Analogous tests may be used to identify whether the polymeric protein comprises a portion that acts as an antigen when administered to the mammalian subject. In such tests, dose, formulation and features of administration of the polymeric proteins are the same to allow a meaningful comparison.

Alternatively, polymeric proteins may be tested in healthy mammalian subjects to determine whether a portion that acts as an antigen is present. Such tests are particularly useful to identify antigen-specific immunostimulation. Suitably, antigen-specific immunostimulation does not occur. Such tests may be useful to confirm non-immunogenicity of the polymeric protein. Components of the polymeric protein that are not naturally found in the mammalian subject to which the protein is to be administered, such as linker peptides, can be tested for immunogenicity and non-immunogenic components selected. In such tests, the polymeric protein is administered and, after an appropriate period of time to allow an immune response to develop against a putative antigen, for example two weeks, a blood sample is tested to determine the level of antibodies directed to the putative antigen, using ELISA.

Suitable animal models may also be used to test for the presence of an antigen portion that causes antigen-specific immunosuppression or a portion that acts as an antigen when administered to the mammalian subject. For example, the mouse model of ITP described in Example 4 may be used. The extent and type of immune response to the antigen is then tested following administration of the polymeric protein lacking the antigen or putative antigen, or polymeric protein comprising the antigen or putative antigen.

Suitably, the polymeric protein for use according to the first aspect of the invention does not activate the classical pathway of complement, although it may be capable of binding to C1q (Mekhaiel et al, 2011a). Complement binding and activation can be assessed by ELISA on wells coated with polymeric protein or control IgG or monomeric Fc as described in Lewis M et al, Mol. Immunol 2008, 45: 818-827, and as performed in Example 3. Wells of 96-well plates were coated overnight with 100µl protein in carbonate buffer, pH 9. Following washing, plates are incubated with 100µl human serum diluted 1/100 in veronal buffered saline containing 0.5mM MgCl₂, 2mM CaCl₂, 0.05% Tween-20, 0.1% gelatin and 0.5% BSA for 1h at room temperature. After washing, plates are incubated with 100µl of either a 1/800 dilution of sheep anti-C1q-HRP (Serotec) or a 1/500 dilution of biotin-conjugated anti-C5b-9 (Quidel, Santa Clara, CA), followed by 100µl streptavidin-HRP (Dako) diluted 1/1000 in PBS-T, 0.5% BSA for 1h at room temperature. Absorbance values below 0.4 are considered negative. In a typical assay for C1q binding, absorbance values above 0.4 are achieved when the plates are coated with polymeric protein of above 2 µg/ml, or above 4 µg/ml or above 10 µg/ml. In a typical assay for C5b-9 deposition, indicative of complement activation, absorbance values above 0.4 are not achieved when the plates are coated with polymeric protein of up to 2 µg/ml, up to 4 µg/ml, up to 10 µg/ml, up to 20 µg/ml or up to 50 µg/ml.

Suitably, the polymeric protein for use according to the first aspect of the invention is capable of binding Protein G or Protein A with sufficient avidity to permit either substrate to be used as a capture agent for purification of the polymeric protein. Protein G binds in the Cγ2-Cγ3 interdomain region and was used to purify the hexameric and monomeric Fc proteins in the Examples. Other receptors which bind to the same region may also bind the polymeric protein, such as TRIM21 (Mallery DL et al, 2010, Proc. Natl. Acad. Sci. U.S.A. 107 (46): 19985-19990; McEwan WA et al 2011, BioEssays. 33: 803-809). TRIM21 is thought to be important in removal of and immunity to viruses.

Suitably, the polymeric protein for use according to the first aspect of the invention has a molecular weight of from about 230 to 400 kD. For example, it may have a molecular weight of about 230, 240, 250, 260, 270, 280, 290, 300, 310, 320, 330, 340, 350, 360, 370, 380, 390 or 400 kD. Polymers within this size range are typically easier for mammalian cells to synthesise and assemble than molecules that have larger molecular weights, like IgM of ∼750kDa, or complex proteins like antibodies that require expression and assembly of two different polypeptide chains (light and heavy chain). Proteins with multiple different polypeptide chains, including antibodies, are more difficult to produce to homogeneity during manufacture.

Suitably, the polymeric protein for use according to the first aspect of the invention has a diameter of about 20 nm, such as from 15 to 25 nm or up to 30 nm. 20 nm particles have been shown to be more readily delivered to lymphatics than 45 and 100 nm nanoparticles, which may be important for function and tissue penetration (Reddy et al, 2007). Smaller particles are also more easily manufactured by recombinant expression technology.

As a consequence of the molecular size and diameter, the polymeric protein typically has a good degree of tissue penetration, which may assist its therapeutic effect (Vollmers & Brandlein, 2006). In comparison with small molecules or monomers, polymers will naturally show a slower penetration over time, but even intact IgM molecules (750 kDa) reach implanted tumours in mice and primary tumours and metastases in patients after *i.v.* or *i.p.* administration (Vollmers et al, 1998a,b, Oncol Rep 5: 549-552; Oncol Rep 5:35-40). It can be demonstrated by standard immunohistochemical techniques that pentameric IgM, when injected *i.p.* can reach and shrink subcutaneously transplanted tumours on the backs of animals (all the Vollmers refs, supra). This shows that molecules as large as 750 kDa are able to leave the peritoneal cavity, enter the circulation and reach implanted tumours. On their way, the molecules have to pass several endothelial barriers of lymphatic and blood vessels before they reach their targets, Jain et al, 2001 J. Control. Release 74: 7-25. Taken together, it is predicted that polymeric proteins for use according to the invention, including the exemplified hexameric Fc protein, will show intermediate penetrance times (hours) between the larger IgM (days) and smaller IgG (minutes). For conditions like ITP requiring a biological activity, an intermediate penetration and accumulation may be an advantage over treatments that use antibodies as carriers where fast penetrance is preferred.

Suitably, the polymeric protein for use according to the first aspect of the invention has a spatial orientation with respect to the cell surface which is cis (each Fc is in the same plane parallel to the cell surface and the long axis of each Fc is perpendicular to the cell surface), rather than trans. The cis orientation permits the ligation of multiple receptors on the same cell, because the receptor binding portions are all closely apposed to the same cell. In contrast, a trans orientation in which the Fcs are in a plane perpendicular to the cell surface might result in cross-linking of two cells by the same protein. In the cis orientation, the biological effects of receptor binding are focussed on a particular cell, and therefore are more likely to be productive.

The polymeric proteins are intended for treatment of autoimmune or inflammatory diseases, according to the first aspect of the invention. "Autoimmune disease" includes any disease in which the immune system attacks the body's own tissues. "Inflammatory disease" includes any disease characterised by a destructive inflammation which may be recurrent or chronic and is not associated with normal tissue repair. Such diseases particularly include "autoinflammatory diseases" in which the innate immune system causes inflammation which may be for unknown reasons. Autoinflammatory disorders are characterized by intense episodes of inflammation that result in such symptoms as fever, rash, or joint swelling. These diseases also carry the risk of amyloidosis, a potentially fatal build up of a blood protein in vital organs.

Suitable autoimmune or inflammatory diseases for treatment include those that are treatable with intravenous immunoglobulin (IVIG). These may be diseases which are currently routinely treated with IVIG or in which IVIG has been found to be clinically useful, such as autoimmune cytopenias, Guillain-Barré syndrome, myasthenia gravis, anti-Factor VIII autoimmune disease, dermatomyositis, vasculitis, and uveitis (See, van der Meche FG et al, Lancet i, 406 (1984); Sultan Y et al, Lancet ii, 765 (1984); Dalakas MC et al, N. Engl. J. Med. 329, 1993 (1993); Jayne DR et al, Lancet 337, 1137 (1991); LeHoang P et al, Ocul. Immunol. Inflamm. 8, 49 (2000). IVIG is typically used to treat idiopathic thrombocytopenic purpura (ITP), Kawasaki disease, Guillain-Barré syndrome and chronic inflammatory demyelinating polyneuropathy (Orange et al, 2006, J Allergy Clin Immunol 117: S525-53). IVIG is also increasingly used to treat a diverse array of other autoimmune diseases which are non-responsive to mainstay therapies, including arthritis, diabetes, myositis, Crohn's colitis and systemic lupus erythematosus.

Autoimmune or inflammatory diseases suitable for treatment include autoimmune cytopenia, idiopathic thrombocytopenic purpura, rheumatoid arthritis, systemic lupus erythematosus, asthma, Kawasaki disease, Guillain-Barré syndrome, Stevens-Johnson syndrome, Crohn's colitis, diabetes, chronic inflammatory demyelinating polyneuropathy myasthenia gravis, anti-Factor VIII autoimmune disease, dermatomyositis, vasculitis, uveitis or Alzheimer's disease.

Conditions to be treated may include an inflammatory disease with an imbalance in cytokine networks, an autoimmune disorder mediated by pathogenic autoantibodies or autoaggressive T cells, or an acute or chronic phase of a chronic relapsing autoimmune, inflammatory, or infectious disease or process. In addition, other medical conditions having an inflammatory component are included, such as Amyotrophic Lateral Sclerosis, Huntington's Disease, Alzheimer's Disease, Parkinson's Disease, Myocardial Infarction, Stroke, Hepatitis B, Hepatitis C, Human Immunodeficiency Virus associated inflammation, adrenoleukodystrophy, and epileptic disorders especially those believed to be associated with postviral encephalitis including Rasmussen Syndrome, West Syndrome, and Lennox-Gastaut Syndrome.

Conditions to be treated may be hematoimmunological diseases, e.g., Idiopathic Thrombocytopenic Purpura, alloimmune/autoimmune thrombocytopenia, Acquired immune thrombocytopenia, Autoimmune neutropenia, Autoimmune hemolytic anemia, Parvovirus B19-associated red cell aplasia, Acquired antifactor VIII autoimmunity, acquired von Willebrand disease, Multiple Myeloma and Monoclonal Gammopathy of Unknown Significance, Aplastic anemia, pure red cell aplasia, Diamond-Blackfan anemia, hemolytic disease of the newborn, Immune-mediated neutropenia, refractoriness to platelet transfusion, neonatal post-transfusion purpura, hemolytic uremic syndrome, systemic Vasculitis, Thrombotic thrombocytopenic purpura, or Evan's syndrome.

Alternatively, a neuroimmunological disease may be treated, e.g., Guillain-Barré syndrome, Chronic Inflammatory Demyelinating Polyradiculoneuropathy, Paraproteinemic IgM demyelinating Polyneuropathy, Lambert-Eaton myasthenic syndrome, Myasthenia gravis, Multifocal Motor Neuropathy, Lower Motor Neuron Syndrome associated with anti-GM1 antibodies, Demyelination, Multiple Sclerosis and optic neuritis, Stiff Man Syndrome, Paraneoplastic cerebellar degeneration with anti-Yo antibodies, paraneoplastic encephalomyelitis, sensory neuropathy with anti-Hu antibodies, epilepsy, Encephalitis, Myelitis, Myelopathy especially associated with Human T-cell lymphotropic virus-1, Autoimmune Diabetic Neuropathy, or Acute Idiopathic Dysautonomic Neuropathy or Alzheimer's disease.

A rheumatic disease may be treated, e.g., Kawasaki's disease, Rheumatoid arthritis, Felty's syndrome, ANCA-positive Vasculitis, Spontaneous Polymyositis, Dermatomyositis, Antiphospholipid syndromes, Recurrent spontaneous abortions, Systemic Lupus Erythematosus, Juvenile idiopathic arthritis, Raynaud's, CREST syndrome or Uveitis.

A dermatoimmunological disease may be treated, e.g., Epidermal Necrolysis, Gangrene, Granuloma, Autoimmune skin blistering diseases including Pemphigus vulgaris, Bullous Pemphigoid, and Pemphigus foliaceus, Vitiligo, Streptococcal toxic shock syndrome, Scleroderma, systemic sclerosis including diffuse and limited cutaneous systemic sclerosis, Atopic dermatitis or steroid dependent Atopic dermatitis.

A musculoskeletal immunological disease may be treated, e.g., Inclusion Body Myositis, Necrotizing fasciitis, Inflammatory Myopathies, Myositis, Anti-Decorin (BJ antigen) Myopathy, Paraneoplastic Necrotic Myopathy, X-linked Vacuolated Myopathy, Penacillamine-induced Polymyositis, Atherosclerosis, Coronary Artery Disease, or Cardiomyopathy.

A gastrointestinal immunological disease may be treated, e.g., pernicious anemia, autoimmune chronic active hepatitis, primary biliary cirrhosis, Celiac disease, dermatitis herpetiformis, cryptogenic cirrhosis, Reactive arthritis, Crohn's disease, Whipple's disease, ulcerative colitis or sclerosing cholangitis.

The disease can be, for example, post-infectious disease inflammation, Asthma, Type 1 Diabetes mellitus with anti-beta cell antibodies, Sjogren's syndrome, Mixed Connective Tissue Disease, Addison's disease, Vogt-Koyanagi-Harada Syndrome, Membranoproliferative glomerulonephritis, Goodpasture's syndrome, Graves' disease, Hashimoto's thyroiditis, Wegener's granulomatosis, micropolyarterits, Churg-Strauss syndrome, Polyarteritis nodosa or Multisystem organ failure.

An exemplary disease for treatment in idiopathic thrombocytopenic purpura (ITP).

Polymeric proteins for use in a given species typically include Fc portions from the IgG of that species, and may also include tailpiece portions from the IgA or IgM of that species. Typically the mammalian subject to be treated is a human, although other mammals and indeed birds, amphibians and reptiles may be treated.

Polymeric proteins are typically provided as appropriately formulated therapeutic compositions. Suitably, they are provided as injectables either as liquid solutions or suspensions; solid forms suitable for solution in, or suspension in, liquid prior to injection may also be prepared. The preparation may also be emulsified. In addition, if desired, minor amounts of auxiliary substances such as wetting or emulsifying agents, pH buffering agents may be included.

The carrier may be preferably a liquid formulation, and is preferably a buffered, isotonic, aqueous solution. Suitably, the therapeutic composition has a pH that is physiologic, or close to physiologic. Suitably it is of physiologic or close to physiologic osmolarity and salinity and/or is sterile and endotoxin free. It may contain sodium chloride and/or sodium acetate. Pharmaceutically acceptable carriers may also include excipients, such as diluents, and the like, and additives, such as stabilizing agents, preservatives, solubilizing agents, and the like. As used herein, the term "pharmaceutically acceptable" means approved by a regulatory agency of US or EU or other government or listed in the U.S. Pharmacopeia or other generally recognized pharmacopeia for use in humans.

Pharmaceutical compositions may be formulated for any appropriate manner of administration, including, for example, topical (e.g., transdermal or ocular), oral, buccal, nasal, vaginal, rectal or parenteral administration. The term parenteral as used herein includes subcutaneous, intradermal, intravascular (e.g., intravenous), intramuscular, spinal, intracranial, intrathecal, intraocular, periocular, intraorbital, intrasynovial and intraperitoneal injection, as well as any similar injection or infusion technique. Forms suitable for oral use include, for example, tablets, troches, lozenges, aqueous or oily suspensions, dispersible powders or granules, emulsion, hard or soft capsules, or syrups or elixirs. Compositions provided herein may be formulated as a lyophilizate. Typically, the composition is formulated for intravenous administration.

Aqueous suspensions contain the active ingredient(s) in admixture with excipients suitable for the manufacture of aqueous suspensions. Such excipients include suspending agents (*e.g*., sodium carboxymethylcellulose, methylcellulose, hydropropylmethylcellulose, sodium alginate, polyvinylpyrrolidone, gum tragacanth and gum acacia); and dispersing or wetting agents (*e.g*., naturally-occurring phosphatides such as lecithin, condensation products of an alkylene oxide with fatty acids such as polyoxyethylene stearate, condensation products of ethylene oxide with long chain aliphatic alcohols such as heptadecaethyleneoxycetanol, condensation products of ethylene oxide with partial esters derived from fatty acids and a hexitol such as polyoxyethylene sorbitol monooleate, or condensation products of ethylene oxide with partial esters derived from fatty acids and hexitol anhydrides such as polyethylene sorbitan monooleate). Aqueous suspensions may also comprise one or more preservatives, for example ethyl, or n-propyl p-hydroxybenzoate, one or more coloring agents, one or more flavoring agents, and one or more sweetening agents, such as sucrose or saccharin.

The formulations may be for local or topical administration, such as for topical application to the skin, wounds or mucous membranes, such as in the eye. Formulations for topical administration typically comprise a topical vehicle combined with active agent(s), with or without additional optional components. Suitable topical vehicles and additional components are well known in the art, and it will be apparent that the choice of a vehicle will depend on the particular physical form and mode of delivery. Topical vehicles include water; organic solvents such as alcohols (*e.g*., ethanol or isopropyl alcohol) or glycerin; glycols (*e.g.,* butylene, isoprene or propylene glycol); aliphatic alcohols (*e.g.,* lanolin); mixtures of water and organic solvents and mixtures of organic solvents such as alcohol and glycerin; lipid-based materials such as fatty acids, acylglycerols (including oils, such as mineral oil, and fats of natural or synthetic origin), phosphoglycerides, sphingolipids and waxes; protein-based materials such as collagen and gelatin; silicone-based materials (both non-volatile and volatile); and hydrocarbon-based materials such as microsponges and polymer matrices.

A pharmaceutical composition may be formulated as inhaled formulations, including sprays, mists, or aerosols. For inhalation formulations, the compounds provided herein may be delivered via any inhalation methods known to those skilled in the art. Such inhalation methods and devices include, but are not limited to, metered dose inhalers with propellants such as CFC or HFA or propellants that are physiologically and environmentally acceptable. Other suitable devices are breath operated inhalers, multidose dry powder inhalers and aerosol nebulizers. Aerosol formulations for use in the subject method typically include propellants, surfactants and co-solvents and may be filled into conventional aerosol containers that are closed by a suitable metering valve.

Inhalant compositions may comprise liquid or powdered compositions containing the active ingredient that are suitable for nebulization and intrabronchial use, or aerosol compositions administered via an aerosol unit dispensing metered doses. Suitable liquid compositions comprise the active ingredient in an aqueous, pharmaceutically acceptable inhalant solvent, *e.g*., isotonic saline or bacteriostatic water. The solutions are administered by means of a pump or squeeze-actuated nebulized spray dispenser, or by any other conventional means for causing or enabling the requisite dosage amount of the liquid composition to be inhaled into the patient's lungs. Suitable formulations, wherein the carrier is a liquid, for administration, as for example, a nasal spray or as nasal drops, include aqueous or oily solutions of the active ingredient.

Formulations or compositions suitable for nasal administration, wherein the carrier is a solid, include a coarse powder having a particle size, for example, in the range of 20 to 500 microns which is administered in the manner in which snuff is administered (*i.e.,* by rapid inhalation through the nasal passage from a container of the powder held close up to the nose). Suitable powder compositions include, by way of illustration, powdered preparations of the active ingredient thoroughly intermixed with lactose or other inert powders acceptable for intrabronchial administration. The powder compositions can be administered via an aerosol dispenser or encased in a breakable capsule which may be inserted by the patient into a device that punctures the capsule and blows the powder out in a steady stream suitable for inhalation.

The actual dosage amount of a composition of the administered to a mammalian subject can be determined by physical and physiological factors such as body weight, severity of condition, the type of disease being treated, previous or concurrent therapeutic interventions, idiopathy of the patient and the route of administration. The practitioner responsible for administration will, in any event, determine the concentration of active ingredient(s) in a composition and appropriate dose(s) for the individual subject.

Pharmaceutical compositions may comprise, for example, at least about 0.1 % of an active compound. In other embodiments, an active compound may comprise between about 2% to about 75% of the weight of the unit, or between about 25% to about 60%, for example, and any range derivable therein. In other non-limiting examples, a dose may also comprise from about 1 milligram/kg/body weight, about 5 milligram/kg/body weight, about 10 milligram/kg/body weight, about 50 milligram/kg/body weight, about 100 milligram/kg/body weight per administration, and any range derivable therein. The effective polymeric protein dose is generally from about 1% to about 20% of the effective IVIG dose. The effective IVIG dose may generally be in the range of about 100 mg/Kg to about 2 grams/Kg, depending on the condition to be treated.

Suitability of polymeric proteins and therapeutic compositions may be tested in animal models, prior to administration to human patients. For an animal model to be suitable, it is important that the Fc receptors in the animal are capable of binding to the Fc receptor binding portions of the polymeric protein. It is known that human immunoglobulins can bind to mouse Fc receptors. For example human IgM binds to the mouse Fcµ-receptor.

Pleass RJ, 2009 Parasite Immunology 31: 529-538 reports which Fc receptors can bind which antibodies from which species. Nevertheless, where the polymeric protein comprises Fc binding portions derived from human immunoglobulin heavy chain sequences, it may be advantageous to use transgenic mice which express human Fc receptors. A suitable transgenic mouse expresses the human FcγRI receptor (CD64) which binds to human IgG1 and IgG3 (Heijnen IA et al, J Clin Invest. 1996 Jan 15;97(2):331-8). Transgenic mice expressing low affinity FcRs are also available, such as FcγRIIA (CD32) (McKenzie SE 2002, Blood Rev 16:3-5).

Suitable mouse models are the mouse model of ITP described in Example 4. Other suitable models include collagen-induced arthritis in mice (Jain *et al,* 2012) or non-obese diabetes (NOD) mouse model (Inoue, Y. et al. (2007) J. Immunol. 179, 764-774).

Conventional recombinant DNA methodologies may be exploited for generating the polymeric proteins, as described, for example in (Sambrook et al, "Molecular cloning: A laboratory manual", 2001, 3rd edition, Cold Spring Harbor Laboratory Press, Cold Spring Harbor, NY). The monomer unit constructs preferably are generated at the DNA level, and the resulting DNAs integrated into expression vectors, and expressed to produce the monomer units which assemble to form the polymeric protein.

A nucleic acid molecule described herein comprises a coding portion which comprises, in a 5' to 3' direction, a coding sequence for an immunoglobulin G heavy chain constant region or a single chain Fc (scFc). The latter is fused in frame with a coding sequence for a tailpiece region. DNA encoding the coding sequences may be in its genomic configuration or its cDNA configuration. It will be appreciated that further coding sequences may be provided between the heavy chain and tailpiece coding sequences, to allow these components to be separated from each other in the expressed protein by linker sequences. Nucleic acids encoding linker sequences may be included, for example, to allow the inclusion of useful restriction sites and/or to allow the heavy chain region and tailpiece coding regions to be transcribed in frame. Suitable coding regions can be amplified by PCR and manipulated using standard techniques (Sambrook *et al,* supra). Mutations compared to native nucleic acid sequences can be made by SOEing PCR or site directed mutagenesis.

Suitably, the coding portion of the nucleic acid molecule encodes a signal peptide, which is contiguous with the polypeptide monomer unit. This facilitates isolation of the expressed monomer units from a host cell. The nucleic acid molecule will therefore comprise a coding portion which comprises, in a 5' to 3' direction, a signal sequence fused in frame with the coding sequence of the monomer unit.

The portion of the DNA encoding the signal sequence preferably encodes a peptide segment which directs the secretion of the monomer unit and thereafter is cleaved away from the remainder of the monomer unit. The signal sequence is a polynucleotide which encodes an amino acid sequence which initiates transport of a protein across the membrane of the endoplasmic reticulum. Signal sequences which are useful include antibody light chain signal sequences, e. g., antibody 14.18 (Gillies et al. (1989) J. OF IMMUNOL. METH., 125: 191), antibody heavy chain signal sequences, e. g., the MOPC141 antibody heavy chain signal sequence (Sakano et al. (1980) NATURE 286: 5774), and any other signal sequences which are known in the art (see, for example, Watson (1984) NUCLEIC ACIDS RESEARCH 12: 5145).

Further aspects of the disclosure are an expression vector comprising the nucleic acid molecule as described above and a host cell comprising the expression vector.

Nucleic acid molecules as described herein may generally be part of expression vectors. As used herein, the term "vector" is understood to mean any nucleic acid comprising a nucleotide sequence competent to be incorporated into a host cell and to be recombined with and integrated into the host cell genome, or to replicate autonomously as an episome. Such vectors include linear nucleic acids, plasmids, phagemids, cosmids, RNA vectors, viral vectors and the like. Non-limiting examples of a viral vector include a retrovirus, an adenovirus and an adeno-associated virus. As used herein, the term "gene expression" or "expression" of monomer unit, is understood to mean the transcription of a DNA sequence, translation of the mRNA transcript, and optionally also secretion of a monomer unit.

Typically, host cells are provided for expression of the expression vector. The cell can be a mammalian, avian, insect, reptilian, bacterial, plant or fungal cell. Examples of mammalian cells include, but are not limited to, human, rabbit, chicken, rodent (e.g. mouse, rat) cells. Typical mammalian cells include a myeloma cell, a Sp2/0 cell, a CHO cell, L cell, COS cell, fibroblast, MDCK cell, HT29 cell, HEK cells, or a T84 cell. A preferred host cell is CHO-K1. Expression vectors may be introduced into host cells using standard techniques, including calcium phosphate transfection, nuclear microinjection, DEAE-dextran transfection, bacterial protoplast fusion and electroporation.

Polymeric proteins may be prepared by methods including (1) preparing a vector comprising the nucleic acid molecule encoding the monomer unit; (2) transfecting a host cell with the vector; (3) culturing the host cell to provide expression; and (4) recovering the polymeric protein.

The polymeric protein may be recovered as products of different molecular sizes. Typically, the desired polymeric protein comprising six monomer units accounts for at least 40% of the total protein composed of monomer units, by weight. Suitably, it accounts for at least 50%, at least 60%, at least 70%, at least 80% or at least 90% of the total protein composed of monomer units, by weight. The high proportion of monomer units that can be recovered as the desired polymeric protein contributes to production efficiency.

If the polymeric protein is secreted by the host cell it can conveniently be recovered by affinity chromatography utilising its affinity for Fc binding agents, such as Protein A or Protein G, suitably Protein-G HiTrap (GE healthcare) columns. Proteins may be eluted from such columns by low pH into neutral buffer. Dialysis may subsequently be performed for buffer exchange. If the host cell does not secrete the polymeric protein, it may be recovered by lysing the cells followed by affinity chromatography.

The present invention will be further illustrated in the following examples, without any limitation thereto.

### Example 1: Production of a recombinant polymeric Fc protein

DNA constructs were prepared as follows. A commercially available pFUSE-hIgG1-Fc2 expression vector was obtained from InvivoGen, sourced via Autogen Bioclear, Wiltshire, UK. The expression vector comprises a coding sequence for a signal sequence from IL2 and, downstream of that, a coding sequence for the Fc portion from human IgG1. To generate a polymeric protein, two changes to the coding sequence of the human IgG1 Fc-portion were made. The 18 amino-acid tailpiece from IgM was sub-cloned onto the C-terminus of the Fc portion, and an additional mutation was made in the Cγ3 domain to convert residues 309 and 310 (EU numbering throughout) to cysteine and leucine respectively.

In order to insert the IgM tailpiece sequence into the commercially available vector, primers were designed which would, when annealed together, form a double stranded sequence with overhanging bases encoding a Nhe1 restriction site to allow subcloning C-terminal to the Fc. In order to maintain the reading frame of the protein encoded by the plasmid, remove an existing stop codon, and allow for convenient restriction sites, an extra DNA base was inserted. The IgM tailpiece sequence is preceded by a short 5' linker which encodes for four amino acids Leu-Val-Leu-Gly; the linker does not affect the function of the IgM tailpiece.

Primers 1 and 2 (SEQ ID No. 1 and 2) were annealed together via a temperature gradient to form the double stranded IgM-tailpiece containing Nhe1 insert.

The pFUSE vector was then digested with the restriction enzyme NheI, and the IgM tailpiece insert above ligated to create an intermediary plasmid. In order to allow the IgM tailpiece to be translated after the Fc region, the stop codon present was mutated in a subsequent step, via site directed mutagenesis, utilising the Quick Change II Kit (Stratagene, La Jolla, CA, USA). Primers 3 and 4 (SEQ ID No. 3 and 4) were designed to remove this stop codon and create an AvrII restriction enzyme site between the Fc region and the IgM tailpiece. This created the plasmid termed pFUSE-hIgG1-Fc-TP.

In human IgM, a cysteine at position 309 is involved in forming a disulphide bridge between two monomers of IgM within the pentamer. In order to better mimic the protein sequence of human IgM, primers 5 & 6 (SEQ ID No. 5 and 6) were designed to introduce a cysteine residue at position 309, again by site-directed mutagenesis, as before. Upon alignment of the nucleotide sequence encoding the protein sequence of human IgM with that of human IgG1-Fc, it was decided to, in addition, replace the neighbouring histidine residue at position 310 with a neutral leucine residue. The final plasmid incorporating both mutations was named pFUSE-hIgG1-Fc-TP-LH309/310CL.

Control plasmids encoding monomers of human IgG1 Fc lacking the 309/310 mutations and the tailpiece were also prepared.

The nucleic acid coding sequence for the monomeric units which assemble into polymers is SEQ ID No. 7.

The coding sequence has the following regions:

| | |
|---|---|
| 1-60 | coding sequence for IL2 signal peptide |
| 61-753 | coding sequence for Fc-region of human IgG1 |
| 325-330 | cys-309 and leu-310 mutations |
| 754-810 | coding seqeunce for IgM tailpiece (including linker region and stop codon) |

The amino acid sequence of the monomeric units which assemble into polymers is SEQ ID No. 8.

The amino acid sequence has the following regions:

| | |
|---|---|
| 1-20 | IL2 signal peptide |
| 21-247 | Fc-region of human IgG1 |
| 109-110 | cys-309 and leu-310 mutations |
| 248-251 | Four amino acid linker |
| 252-269 | IgM tailpiece |

During expression, the IL2 signal peptide is cleaved off and so the final protein product has 249 amino acids.

Polymeric Fc proteins and control monomeric Fc proteins were prepared as follows. CHO-K1 cells (European Collection of Cell Cultures) were transfected by electroporation with plasmids and positive clones selected. The cells were grown in DMEM complete media supplemented with 10% ultra-low bovine IgG FCS, 100 IU/ml penicillin, and 100 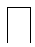 µgml⁻¹ streptomycin (PAA) at 37°C/5%CO₂. Stable transfectants were selected in medium containing 400 µgml⁻¹ of Zeocin (Invivogen). Clones secreting Fc-fusion proteins were detected by sandwich enzyme-linked immunosorbent assay (ELISA) using goat anti-human IgG-Fc (Sigma-Aldrich: A0170). From large-scale cultures in DMEM supplemented with ultralow IgG containing FBS (Gibco), Fc-fusion proteins were purified on Protein-G-Sepharose (GE Healthcare, Little Chalfont, Bucks, UK). Eluted fractions from the affinity purification were pooled and separated by size-exclusion chromatography on a high-performance Superdex-200 10/300GL column using an AKTA_{FPLC} (GE Healthcare). Eluted fractions were compared against known high MW gel filtration standards (Biorad). The integrity of the proteins was verified by SDS-PAGE on native 6% Tris-glycine gels against SeeBlue2 pre-stained molecular weight markers (Novex-Invitrogen).

Polymeric proteins were expressed as complexes of about 312 kD and about 100 kD, consistent with expression as hexameric and dimeric entities. The proportion was about 90% hexameric versus 10% dimeric (w/w) according to size-exclusion chromatography, as illustrated in Figure 1B.

### Example 2: Structural characterisation of a recombinant polymeric Fc protein

Hexameric-Fc were imaged by tapping mode atomic force microscopy (AFM) under solution. Hexameric-Fc form a highly uniform population of well defined structures. The obtained AFM images showed the complex to be cylindrical in structure (18±2nm (n=51) in diameter, 5.7±0.4nm (n=54) in height). Representative images are shown in Figure 1A. The obtained images are also consistent with dimensions predicted from *in silico* modelling analysis, also illustrated in Figure 1A.

AFM was performed as follows, and as described in Mekhaiel et al, 2011a. Stock solutions of hIgG1-Fc-LH309/310CL-TP in 1xHBSS buffer were diluted to 10 µgml⁻¹ in 0.2xHBSS buffer and then directly applied to a freshly cleaved fragment of muscovite mica. After incubating for 20 minutes, the sample was rinsed extensively with 0.2xHBSS buffer to remove unadsorbed molecules. The samples were always under solution during transport to and imaging within the AFM. Imagaing was performed in the tapping mode with a Nanoscope IIIa Multimode AFM (Veeco, Santa Barbara, CA) using silicon nitride cantilevers (NP-S, Veeco Probes, Santa Barbara, CA) with a spring constant of 0.32N/m in 0.5xHBSS buffer. The typical scan rate and initial oscillation amplitude were 3Hz and 20nm, respectively, and the applied force was minimized to ∼0.1nN. The piezoscanner (9µm, D scanner, Veeco) was calibrated using mica and gold ruling. All images reported were reproducible with different tips and different fast-scan directions. All lateral dimensions were determined from the full-width at half height.

Molecular (*in silico*) modelling was performed as follows, and as described in Mekhaiel et al, 2011a. The monomer was constructed from the crystal structure of the hIgG1 Fc-domain, using a random chain structure for both the hinge and tailpiece regions. Each monomer was energy minimized before being assembled into the hexameric complex. All energy calculations were performed using VMD/NAMD using the CHARMM27 force field. NAMD was developed by the Theoretical Biophysics Group in the Beckman Institute for Advanced Science and Technology at the University of Illinois at Urbana-Champaign. To construct the hexamer, two experimental observations were taken into account: first, the stoichiometry of the complex is finite (that is, oligomerization does not proceed indefinitely), and second, oligomerization required the Cys309 mutation. The former observation suggests that there is some physical reason preventing further assembly, which we reasoned was owing to the complex being ring-shaped, similar to those seen with natural IgM complexes. The latter observation suggests that adjacent monomers are linked through these C309 residues. For these linkages to be possible in a ring-shaped complex, these residues must all lie within a common plane, as is the case with the homologous cysteine residues in the IgM pentamer. With these Fc-fusions, there are two types of oligomers that can satisfy these criteria: star-shaped, in which the C309 plane is the same as the Fc-plane, and barrel-shaped, where the C309 plane is perpendicular to the Fc-plane. Both models were examined to determine which produced a structure with lower energy, subject to one additional criterion. Since these Fc-fusions have not evolved to interact with each other but rather link by virtue of the mutated C309 residues, we assumed that the monomers do not undergo significant structural changes to form these bonds, and so assigned an energetic penalty to any such changes. This was achieved by constraining the backbone atoms within a harmonic well (spring constant, 1 kcal/mol/Å²) centered on the positions of the energy-minimized monomeric structures. By monitoring the contribution of this constraining potential to the total minimized energy, the extent to which each monomer changed conformation from the monomeric form could be evaluated. Without this constraint, the star-shaped and barrel-shaped structures, evaluated at a range of initial C309-C309 distances, were of a similar minimized energy. However with the constraint, at all distances, it was clear that the barrel-shaped structure required significantly fewer structural changes than the star-shaped model to form the C309-C309 disulfide bridge. Thus, without the constraint, the barrel-shaped structure achieves a similar minimized energy as the star-shaped structure, but requiring fewer changes to the monomeric form. We therefore favor a barrel-shaped structure for the hexamer. The final model was constructed at the closest initial C309-C309 distance at which there was the smallest degree of structural changes to the monomeric structure. These calculations were performed without any of the tailpieces connected to other monomers. With this minimized model, the tailpieces were connected to other, randomly selected, monomers within the complex, and then the entire complex was solvated with TIP3 water, minimized, and finally equilibrated, as judged by the root-mean-squared-deviation of the protein backbone.

### Example 3: Interaction of a polymeric Fc protein with components of the immune system

### 1. Interactions with Fc-receptors

Hexameric Fc bound to all of the Fc receptors tested, namely human FcγRI, FcγRIIA and FcγRIIB, and mouse FcγRI and FcγRIIB. The hexameric-Fc bound with higher affinity (in the nanomolar range) to the low affinity human FcγRs (FcγRIIA^{R131} and FcγRIIB) than dimers or monomers that bind to the same receptors in the micromolar range. This confirms that higher order polymers do have improved binding kinetics to receptors known to be involved in protecting from ITP. In SPR analysis, Hexameric-Fc bound to the human FcγRI with a KA (1/M) of 1.6x10¹⁰. Dimers bound with a KA (1/M) of 6.4x10⁹. Binding of hexameric or monomeric Fc to Fc receptors by ELISA is shown in Figure 2.

Another receptor that may play a role in ITP is FcγRIII (Park-Min et al, 2007). Hexameric-Fc can be expected to bind human FcγRIII because the binding site for FcγRIII on IgG overlaps with that of FcγRIIB and FcγRI (Sondermann et al, 2001). The IgG lower hinge region plays a key role in the interaction with FcγRI and FcγRII, and it seems probable that all of the FcγRs share a common mode of interaction with IgG (Woof & Burton, 2004). Indeed, it has been possible to produce model complexes between IgG Fc and FcγRI and FcγRII based on the FcγRIII-IgG Fc crystal structure and thereby rationalize particular binding characteristics (Sondermann et al, 2001). The interaction of FcγRIII and hexameric-Fc complex was modelled *in silico* using the crystal structure of FcγRIII. The model shows that the binding site in the hexamer for FcγRIII is most likely exposed, as it clearly is for the other FcyRs. We therefore predict that FcγRIII would bind to the hexameric-Fc.

Binding of Fc proteins to Fc receptors was determined by enzyme linked immunosorbent assays (ELISA) as described below and in Mekhaiel et al, 2011a. Microtiter wells (Nunc) were coated with titrated amounts of the Fc proteins (50-0.3 nM) in PBS or carbonate buffer pH9 and incubated over night at 4 °C prior to blocking with 4% skimmed milk (Acumedia) for 1 h at room temperature (RT). The wells were washed four times with PBS/0.005% Tween 20 (PBS/T) pH 7.4 before addition of GST or HIS-fused hFcγRI, hFcγRIIA, hFcγRIIB, mFcγRI or mFcγRIIB diluted in 4% skimmed milk PBS/0.005% Tween 20 (PBS/T) pH 7.4 and added to the wells. After incubation for 2 h at RT and washing as above, an HRP-conjugated polyclonal anti-GST from goat (1:8000; GE Healthcare) was added and incubated for 1 h at RT. Wells were washed as above, 100 µl of the substrate TMB (Calbiochem) was added to each well and incubated for 45 minutes before 100 µl of 0.25 M HCl was added. The absorbance was measured at 450 nm using a Sunrise TECAN spectrophotometer (TECAN, Maennedorf, Switzerland).

Binding to human FcγRI was also analysed by surface plasmon resonance analysis, exactly as described below in relation to FcRn.

### 2. Interactions with glycan receptors known to play a role in controlling autoimmunity

Human DC-SIGN and its homologue in mice (SIGN-R1) have been shown to be involved in controlling ITP by binding the terminal sialic acid found on the glycan attached at N297 of the Fc of IgG (Anthony et al, 2011; Samuelsson et al, 2001). Sialylation of hexameric-Fc protein was demonstrated as follows. Reduced proteins were run on 4-12% Bis-Tris gels and transferred to PVDF membranes and blocked with 1% Roche blocking reagent as described previously (Samuelsson et al, 2001). Membranes were then incubated with a 1/200 dilution of biotinylated *Sambucus nigra* bark lectin (Vector laboratories) and developed with streptavidin-HRP (Serotec). *In silico* modelling predicts that these terminal sialic acids found on hexameric-Fc proteins would be available to bind DC-SIGN or SIGN-R1 (Figure 1).

### 3. Interactions with newly described receptors that may play a role in controlling autoimmune disease

Human B cell inhibitory receptors are important in controlling autoimmune disease (Pritchard & Smith, 2003). Two inhibitory receptors for IgG have been described on the surface of human B cells: FcγRIIb (Daeron et al, 1995) and more recently FcRL5 (Wilson et al, 2012). Although it is possible that FcRL5 is redundant with FcγRIIb in human B cells, the potential for simultaneous recruitment of SHIP-1 by FcγRIIb and SHP-1 to FcRL5 provides a substantial barrier to recurrent activation of B cells. Intriguingly, FcRL5 on innate B cells is also targeted by Poxvirus MHC Class I-like immunoevasins, suggesting an important role for FcRL5 in regulating immunity (Campbell et al, 2010). FcRL5 only binds complexed IgG and not monomeric IgG and therefore IVIG would be less likely to bind this receptor with high avidity, although the polymeric fraction of IVIG might bind with high avidity. Taken together, FcγRIIb and FcRL5 may limit B cell activation against chronic pathogens or self-reactive antigen, and approaches that have the potential to target both receptors may prove beneficial in therapies aimed at controlling B cell activation.

We therefore investigated the binding of hexameric-IgG1-Fc to human CD19+ B cells (Figure 3). As a first step to evaluate the interaction of hexameric IgG1-Fc with immune cells involved in controlling autoimmunity, we investigated the ability of hexameric IgG1-Fc to bind human B cells by FACS analysis. We show that hexameric IgG1-Fc bound to CD19⁺ B cells purified from the peripheral circulation of healthy human volunteers. Flow cytometry was performed as described in Mekhaiel et al, 2011a. Human leucocytes were purified from whole blood by centrifugation on Polymorphoprep gradients according to manufacturer's instructions. 1 x 10⁵ cells were incubated with 200 µl FACs buffer (phosphate-buffered saline, 0.2% bovine serum albumin, 5% goat serum) containing 50 µg of hexa-Fc or buffer only for 1h at room temperature. Cells were washed twice with FACs buffer and incubated for 1h at 4°C with 1/500 dilution of F(ab')₂ goat anti-hIgG-Fc-phycoerythrin (PE) and goat anti-hCD19-fluorescein isothiocyanate (FITC)-conjugated Abs (Southern Biotechnology) in 200 µl FACs buffer. After washing with FACs buffer, cells were analyzed on a FACScan (BD Biosciences). Data acquisition was conducted with CELLQuest software (BD Biosciences) and the analysis performed with FlowJo version 9.1. CD19+ B lymphocytes were gated taking into account their forward and side scatter profiles, and binding of Fc protein was detected with the PE-labelled secondary antibody. No binding of the PE-labelled secondary antibody was detected in the absence of the Fc protein. That hexamer-Fc was binding FcRL5 on the surface of human B cells was demonstrated by prior incubation of cells with FcRL5 specific blocking monoclonal antibody 509F6, which ablated binding of the hexameric Fc protein.

### 4. Binding to the neonatal FcRn

We investigated the ability of hexameric-Fc to interact with neonatal FcRn, responsible for maintaining the long half-life of Abs in the circulation (Mekhaiel et al, 2011a). Although FcRn does not play a dominant role in ITP, it may play a role in hexameric-Fc mediated control of more chronic autoimmune disease, where a long half-life for the therapy is important. Although hexameric-Fc was unable to bind human FcRn it did bind with nM affinity to mouse FcRn (0.1-10 nM at pH6) in line with previous observation (Andersen et al, 2010). This suggests that minor modifications to the existing construct e.g. reversion of Leu310 to His310 may reinstate binding to human FcRn.

Binding of Fc proteins to FcRn was determined by surface plasmon resonance analysis and as described below and in Mekhaiel et al, 2011a. Surface Plasmon Resonance (SPR) analyses were carried out using a Biacore 3000 instrument (GE Healthcare). Flow cells of CM5 sensor chips were coupled with recombinant forms of soluble human or mouse FcRn (hFcRn or mFcRn; ∼ 2200-3000 RU) using amine coupling chemistry as described in the protocol provided by the manufacturer. The coupling was performed by injecting 2 µgml⁻¹ of the protein in 10 mM sodium acetate pH 5.0 (GE healthcare). Phosphate buffer (67 mM phosphate buffer, 0.15 M NaCl, 0.005% Tween 20) at pH 6.0, or HBS-EP buffer (0.01 M HEPES, 0.15 M NaCl, 3 mM EDTA, 0.005% surfactant P20) was used as running buffer and dilution buffer. 100 nM of each of the Fc-fusion proteins were injected over immobilized receptor at pH 6.0 or pH 7.4, with a flow rate of 20 µl/min at 25 °C. Regeneration of the surfaces was done using injections of HBS-EP buffer at pH 7.4 (GE Healthcare). In all experiments, data were zero adjusted and the reference cell subtracted. Data were evaluated using the BIAevaluation 4.1 software (GE Healthcare).

### 5. Binding to complement

Most Fc-fusions studied to date do not bind C1 q and are unable to activate the ensuing complement cascade (Czajkowsky et al, 2012). This inability of Fc-fusions to activate complement likely stems from the lack of Fab residues in the fusion that contribute to interactions of intact IgG with C1 q (Gaboriaud et al, 2003). Because Hexameric-Fc lacks Fab residues it is unlikely to be able to bind C1q with the same affinity seen with native antibodies. Nonetheless we did investigate the ability of hexameric-Fc to bind C1 q and activate the classical pathway (Mekhaiel et al, 2011a). Hexameric-Fc bound C1q poorly compared to dimers, which in turn bound less well than intact monomeric IgG, a gradation reflected in commensurate decreases in detection of the C5-9 terminal complex. There was also a complete lack of an Arthus reaction, a type III hypersensitivity reaction initiated by immune-complex formation at the site of inoculation, even when these reagents were administered to mice *i.p.* or when administered s.c. together with alum, observations made at the time of injection. The lack of complement activation is generally considered a desirable property of injected therapies aimed at treating autoimmune disease. However, C1q binding without subsequent activation of the classical pathway may be advantageous in light of the finding that DC-SIGN, C1q, and gC1qR form a trimolecular receptor complex on the surface of monocyte-derived immature dendritic cells that may be important in controlling autoimmune disease (Hosszu et al, Blood 2012; 120: 1228-1236).

The Arthus reaction involves the *in situ* formation of antigen/antibody complexes after the intradermal injection of an antigen (as seen in passive immunity). If the animal/patient was previously sensitized (has circulating antibody), an Arthus reaction occurs. Typical of most mechanisms of the type III hypersensitivity, Arthus manifests as local vasculitis due to deposition of IgG-based immune complexes in dermal blood vessels. Activation of complement primarily results in cleavage of soluble anaphylotoxins C5a and C3a, which drive recruitment of PMNs as well as local mast cell degranulation (requiring the binding of the immune complex onto FcγRIII), resulting in an inflammatory response. Further aggregation of immune complex-related processes ensures in the tissue vessel walls a local fibrinoid necrosis with ischemia-aggravating superimposed thrombosis. The end result is a localized area of redness, readily observable by eye at the site of injection that in duration typically lasts a day or so.

### Example 4: Hexameric-Fc can restore platelet counts in a clinically relevant mouse model of ITP.

In a commonly used platelet depletion model for ITP we could show that as little as 0.035g/kg of hexameric Fc had a significant positive effect on platelet recovery by comparison with a similar dose 0.035g/kg of IVIG.

Balb/C mice were injected *i.p.* with Hexa-Fc at 0.035g/kg, IVIG at 0.035g/kg or 2g/kg (GammaGard), or PBS in a final volume of 200 µl. One hour later ITP was induced in all mice by *i.p.* injection of 12.5µg IgG1-κ specific for mouse integrin β₃ chain/CD61, clone 2C9.G2 (BD Pharmingen; Product #: 550541) in a 200µl volume of sterile PBS. Platelets were enumerated at 12h, 24h, 36h and 48h time-points post treatment by flow cytometry using an APC-conjugated anti-CD61 antibody. n = 4 mice per group from 2 independent experiments. Group means ±SD were analyzed by repeated ANOVA using Dunnett's multiple comparison test versus the control group. Control vs hexamer: q=3.13,*P<0.05; control vs IVIG: q=8.66,***P<0.0001. Data are mean % platelets ±SD. Mean % platelet counts in each group prior to depletion: hexamer group = 9.6%±1.2, high dose IVIG group = 9.4%±1.2, low dose IVIG group = 9.4%±1.2, PBS group = 9.3%±1.2.

### Example 5: Treating patients with ITP

Treatment protocols for ITP with the disclosed hexameric Fc protein would be utilized in a manner tracking standard guidelines for ITP hIVIG therapy such as the Executive Committee of the American Society of Hematology practice guideline for the diagnosis and management of primary immune thrombocytopenic purpura. See George, J N, et al. Idiopathic thrombocytopenic purpura: a practice guideline developed by explicit methods for the American Society of Hematology. Blood. 1996 Jul. 1; 88(1):3-40. Alternatively, the protocols for ITP may include an initial administration phase with dosages of about 0.1 to about 0.001 times the above treatment protocol dosages. The initial low dose phase is designed to minimize any short term pro-inflammatory effects of the Fc protein administration while still being sufficient to induce a long term anti-inflammatory effect, which is subsequently enhanced and maintained by the second phase standard dosing described above. The rationale for this alternative approach is that some polymeric proteins may have both a short term inflammatory effect as well as a long term anti-inflammatory effect through decreasing the expression of FcγRIIa. An initial low dose (or initial low doses) can be used to stimulate the long term anti-inflammatory effect while minimizing the short term inflammatory effect.

The Fc protein would be administered intravenously and the effective Fc protein dose is generally from about 1% to about 20% of the effective IVIG dose. The effective hIVIG dose in ITP is generally in the range of about 100 mg/Kg to about 2 grams/Kg administered every 10-21 days.

The Fc protein intravenous formulation will be substantially the same as FDA approved hIVIG formulations but may exclude the stabilizers present in some hIVIG formulations.

### Example 6: In silico modelling of Fc proteins

The ability of Fc proteins to modulate autoimmune or inflammatory diseases will depend on their ability to interact with appropriate components of the immune system, whilst not provoking unwanted reactions. This in turn will depend on the structure of the Fc protein. In the case of oligomers, tertiary and quaternary structure will have an important impact on the availability of protein surfaces and exposed moieties for binding to components of the immune system. A uniform and defined tertiary and quaternary structure may reduce the risk of unwanted reactions which might otherwise be provoked by minority species within complex mixtures. A uniform and defined structure also permits easier quality control of medicinal products.

The inventor has shown that hexameric-Fc disclosed herein has a regular and uniform cylindrical shape (Example 2), binds to various desirable receptors and components of the immune system (Example 3) and does not activate complement (Example 3). These findings are all consistent with the *in silico* modelling of hexameric-Fc described in Example 1.

US 2010/0239633 (University of Maryland, Baltimore; Strome) discloses IVIG replacement compounds comprising multiple linked Fc portions. Linear or pentameric arrangements are envisaged. Pentamerization is envisaged by the addition of the Cµ4 domain of IgM and the J chain (as depicted in Figs 10A-D and on page 18 preferred embodiments of US2010/0239633). In native IgM, the Cµ4 domain of the heavy chain constant region binds to the J chain to effect pentamerization. Extensive *in silico* modelling/molecular dynamics simulations were performed to determine if the structures proposed in US2010/0239633 are viable.

In the structures depicted in Fig 10A-D of US2010/0239633, a Cµ4 domain is linked via its N-terminus to the C-terminus of a Cγ3 domain (i.e. the CH3 domain of an IgG molecule), and the Cγ3 domain is linked to a Cγ2 domain. Like domains of the monomers are depicted as dimerizing together to form monomer arms of the higher order structure. However, this presents a conformational problem for the structures.

In native immunoglobulins, the Cµ3, Cµ4, Cγ2 and Cγ3 domains form homodimers which are 'V'-shaped, or inverted 'V'-shaped, i.e '/\'. One monomer forms the '\' part of the 'V' and a like monomer forms the '/' part of the 'V'; or one monomer forms the '/' part of the 'A' and a like monomer forms the '\' part of the '/\'. Cγ3 or Cµ4 dimers are 'V'-shaped whereas Cµ3 and Cγ2 are '/\'-shaped. In a native immunoglobulin, 'V' and '/\'-shaped dimers alternate. For example, the N-termini of the 'V'-shaped Cγ3 domains are positioned to be contiguous with the C-termini of the '/\'-shaped Cγ2. Likewise, the N-termini of the 'V'-shaped Cµ4 domains are positioned to be contiguous with the C-termini of the'/\'-shaped Cµ3 domains.

In the monomers depicted in US2010/0239633, the 'V'-shaped Cγ3 domain is linked directly to another 'V'-shaped Cµ4 domain. This is a problem since the C-terminal residues of the Cγ3 are close to each other whereas the N-term residues of the Cµ4 are far from each other. It is likely that a linker would be required to join the domains together. The most likely result of this structural limitation is that, for the linker lengths that would most likely be employed, the Cµ4 domains would have to be rotated by 90 degrees with respect to the Cγ2-Cγ3 domains. The Cµ4 domain would be under rotational strain. The intra-monomer disulphide linkages within the Cγ3s and the intra-monomer disulphide linkages within the Cµ4s are in competition with each other. One or both will be weakened. Therefore, the protein might be unstable or might not fold properly.

To mimic the normal orientation of the Cγ2-Cγ3 domains with respect to the Cµ4 domain as is found in Cγ2-Cγ3-Cµ4, domains of IgM would require linkers in excess of 20 amino acids. The introduction of linkers (unspecified sequences) raises significant problems for these structures, not least of which is the increased likelihood of unwanted immunogenicity. There would also be a negative impact on manufacturability and increased likelihood of proteolytic cleavage. This geometric limitation of the Cγ3-Cµ4 linkage has significant consequences for the oligomer that make it fundamentally different from the hexameric-Fc protein.

The linker sequences between the Fcγ and Fcµ domains are critical. However, although the term 'linker' connotes flexibility, there would be limitations to this flexibility in the present case. With a single point connection, such as between a Fab and an Fc region (at the hinge), there can indeed be a great deal of rotational flexibility. The difference in the embodiments as depicted in US2010/0239633 is that there are two places of linkage, one between each Cγ3-Cµ4 pair. So rotations of one Fc relative to the other Fc results in a twisting together of the two linkers, which is energetically unfavourable, increasing the risk of mis-folding or instability.

It is improbable that the structures described in US2010/0239633 would form well-defined oligomers (as in IgM) with their linkages only by the Cµ4 domain. The 18 amino-acid C-terminal tails found in all polymeric antibodies, being unstructured, cannot be responsible for the regular pentameric and/or hexameric structure - that must come from the presence of a structured Ig-domain(s) within the Fc. With polymerization occurring through the structured Ig regions, the stoichiometry is set by the size and shape of the structured regions. Interactions between random unstructured regions must necessarily lead to a wide range of polymeric sizes. So, to determine whether the (few) inter-monomer contacts in the Cµ4 domains observed in IgM would be sufficient to maintain the pentameric stoichiometry, we undertook extensive molecular dynamics simulations of just these structured regions (i.e. lacking tailpieces). During simulations, the structured regions of Cµ4 quickly lose their pentameric arrangement, and begin to (apparently) clump together (non-specifically). A regular pentameric/hexameric structure is thus very improbable. This is to be contrasted with native IgM, in which both the Cµ4 and Cµ3 domains contribute to the regular pentameric/hexameric structure. Although US2010/0239633 suggests using the J-chain to facilitate polymerisation, this would not be expected to be successful. The J-chain can stabilize immunoglobulin structures, but cannot convert a monomer or dimer into a higher order oligomer. For example, the J-chain can facilitate dimerisation of IgA, but it is not sufficient to form higher order oligomers as in IgM, because the Ig domains of IgA are not suitable for higher order polymerisation.

The most likely structure that could possibly form if polymerization could occur through the random tails is a 3d star-shaped structure, where the tails are located within the central corona and the Fc-regions of the monomers project out radially, in 3d. With polymerization occurring through unstructured peptides, there can be no well-defined stoichiometry and whatever can fit in three dimensions will occur. There is the possibility of dimers up to probably 20-mers depending on how many linking tails can fit inside the central sphere of interacting tails.

It is also important to note that experimentally, we have found that the addition of the 18 amino-acid tailpiece to the Fc of human IgG1 is insufficient to induce multimerization (Mekhaiel, 2011a). We have also shown that with constant domain swaps, the presence of the Cµ4 domain is unable to induce pentamer and/or hexamer formation (Ghumra et al, 2009). Thus, we consider the possibility that the structures in US2010/0239633 can actually polymerize, whether by Cµ4 domains or by the tails, as unlikely.

Even if the predictions arising from the models are wrong and the Cµ4 domains in the monomers are able to interact to form structures similar to IgM, the resulting structures would be very different to the hexameric-Fc. Assuming that the Cµ4 interact as they do in IgM (which is assumed by US2010/0239633), the need for the Cµ4 domains to be rotated by 90 degrees with respect to the Cγ2-Cγ3 domains would cause the Cγ2-Cγ3 regions to (largely) 'face' each other in any oligomer which was able to form from the monomers. That is, the side of the Cγ2-Cγ3 would directly face the opposing side of the Cγ2-Cγ3 in the neighbouring arm. The overall structure would resemble a closed umbrella. In contrast, in the hexameric-Fc protein, these sides form the walls of the 'barrel' and thus do not face each other, but rather face outwards. Receptors that can easily bind to these sides in the hexameric-Fc protein might thus not bind to the US2010/0239633 oligomers. The sugared regions in the Cγ2-Cγ3 Fc unit are critical to binding by both Fc- and glycan-receptors. In the hexameric-Fc, the sugared regions face out, so receptors that bind to the sugared regions can bind. In contrast, in the US2010/0239633 oligomers, the receptors for these glycans would have to get in between the monomers, and although not impossible, this would nonetheless impact on overall binding strengths between the oligomer and critical receptors. While a single monomer of the US2010/0239633 oligomer might be orientated so that a single pair of Cγ2-Cγ3 domains is accessible to receptors, it is not clear whether more than one other monomer in the oligomer could bind to a different receptor on the same surface, owing to geometric considerations described above and to the physical size of the monomers. Thus, it is not clear whether the US2010/0239633 oligomer could bind to more than one/two receptors simultaneously. Similar issues would arise from the use of alternative polymerizing constant domains e.g. those from IgA. Thus, despite the presence of multiple monomer units in the structures envisaged in US2010/0239633, the binding to critical Fc receptors and other components of the immune system might be no more avid than is observed with monomeric or dimeric immunoglobulins, if such structures can form at all.

This building block strategy seriously limits the interaction possibilities of the structures described in US2010/0239633. More problematic is that even if these molecules can be shown to multimerize, the presence of the Cµ4 domain will have serious impact on the bio-distribution and *in vivo* interactions possible from these chimeric molecules that are expected to be significantly different to those seen with hexameric-Fc as described in this application. These are summarized in detail in **Table 1.**

**Table 1: Differences between Hexameric-Fc and structures envisaged in US2010/0239633**

| | **Hexameric-Fc** | **Core structure (Cµ4 based) (Fig 10A & C)** |
|---|---|---|
| Structure | Circular hexameric barrel (evidenced by AFM and SEC) | Anticipated 2D star as in IgM, if it would polymerise at all |
| Molecular weight | 312 kDa | -625 kDa predicted from sequence. A molecule as large as this may be difficult for mammalian cells to synthesize for manufacturing purposes. |
| Diameter* * impacts on tissue penetration | 20 nm. 20 nm particles have been shown to be more readily delivered to lymphatics than 45 and 100 nm nanoparticles. Important for function and tissue penetration (Reddy et al, 2007). | 50 - 60 nm (size of small virus) |
| Spatial orientation with respect to cell surface | Cis (long-axis perpendicular to cell surface). Predicted to be better at clustering receptors in lipid rafts on the same cell. | Trans (potential of cross-linking receptors on different cells) |
| Valency | Hexamers | Unknown (Pentamer?) |
| Glycans and sialic acid present | Yes | Unknown |
| Requirement for J chain interactions for multimerization | No | Envisaged. If J chain is required, this complicates manufacture significantly. |
| Increased binding to classical Fcg-receptors | Yes to FcgRI, FcgRIIA^{H131}, FcgRIIA^{R131}, FcgRIIB (low nM affinity either observed directly or predicted from modelling) | Not known |
| Increased binding to FcRL5 | Yes (low nM affinity) | Unknown |
| Increased binding to FcµR | No binding seen | Likely if Cµ3 domains included, binding site for receptor located in Cµ3-Cµ4 domain boundary (Kubagawa et al, 2009) |
| Increased binding to Fcα/µR | No binding seen | Possible, binding site for receptor located at Cµ3-Cµ4 boundary domain (Ghumra et al, 2009) |
| Binding to human FcRn | Weak (high µM affinity) | Not known |
| Binding to mouse FcRn | Yes (low nM affinity) | Not known |
| Binding to protein G/A required for purification and manufacture | Yes (low nM affinity) | Not known |
| Activation of complement cascade | No | C1q binds in the Cµ3 domain (Czajkowsky & Shao, 2009). Since IgM is 1,000 fold better at activating the complement cascade than IgG this is a serious potential concern with structures containing IgM domains other than Cµ4 |
| Likely immunogenic | No, initial evidence suggests they are non-immunogenic | Unknown |
| Interactions with glycan receptors | Unknown | Unknown |
| Tissue penetration | Intermediate, slower than smaller IgG molecules (∼150 kDa) but faster than the larger IgM (-750 kDa) and therefore predicted to reach lymphoid organs faster. | Slow due to excessively large size. Therefore predicted to reach lymphoid organs more slowly when injected |
| Can reverse ITP in mouse model of platelet loss in vivo | Yes, -50 fold improved over IVIG | Unknown |

### References

Debré M et al. Lancet 342, 945-9 (1993)
Samuelsson, A., Towers, T.L. & Ravetch, J.V. Science 291, 484-6 (2001)
Bazin, R. et al. Br J Haematol 135, 97-100 (2006)
Crow. A.R. et al. Blood 102, 558-60 (2003)
Clynes, R. et al J. Clin. Invest. 115: 25-7 (2005).
Leontyev D et al. Blood 119: 5261-5264 (2012)
Araujo, L.M. et al. J Immunol 186, 3289-93 (2011)
Roopenian DC, Akilesh S (2007) Nature reviews Immunology 7: 715-725
Crow AR et al (2011) Blood 118: 6403-6406
Machino Y et al. Clin Exp Immunol. 162, 415-24 (2010).
Anthony RM et a. Proc Natl Acad Sci USA 105, 19571-78 (2008).
Séïté, J.F. et al. Blood 116, 1698-704 (2010).
Anthony RM et al Nature 475, 110-116 (2011).
Mekhaiel DN et al (2011 a). Scientific reports 1: 124
Mekhaiel DN et al (2011 b) Trends in parasitology 27: 523-529
Machino Y et al (2012) Biochem Biophys Res Comm*un*
Roux KH, Tankersley DL (1990) J Immunol 144: 1387-1395
Teeling JL et al (2001) Blood 98: 1095-1099
Yoo EM et al (2003) J Immunol 170: 3134-3138
Ballow M (2011) The Journal of allergy and clinical immunology 127: 315-323
Jain et al (2012) Arthritis Research & Therapy 14:R192
Zhang Y et al (2011) European journal of immunology 41: 1154-1164
Reddy ST et al (2007) Nature biotechnology 25: 1159-1164
Vollmers HP, Brandlein S (2006) Histology and histopathology 21: 1355-1366
Park-Min KH et al (2007) Immunity 26: 67-78
Sondermann P, Kaiser J, Jacob U (2001) Journal of molecular biology 309: 737-749
Pritchard NR, Smith KG (2003) Immunology 108: 263-273
Daeron M et al (1995) The Journal of clinical investigation 95: 577-585
Wilson TJ, Fuchs A, Colonna M (2012) J Immunol:188
Campbell JA et al (2010) J Immunol 185: 28-32
Bruhns P et al (2009) Blood 113: 3716-3725
Nimmerjahn F, Ravetch JV (2008) Nature Reviews Immunology 8: 34-47
Nimmerjahn & Ravetch (2007) J. Exp. Med 204: 11-15
Davis RS (2007) Annual review of immunology 25: 525-560
Dement-Brown J et al (2012) Journal of leukocyte biology 91: 59-67
Ise T et al (2005) Clinical cancer research : an official journal of the American
Association for Cancer Research 11: 87-96
Czajkowsky DM et al (2012) EMBO molecular medicine
Ghumra A et al (2009) European journal of immunology 39: 1147-1156
Ghumra A et al (2008) Journal of Immunology 181: 1988-2000
Czajkowsky DM, Shao Z (2009) Proc Natl Acad Sci USA 106: 14960-14965
Kubagawa H et al (2009) The Journal of experimental medicine 206: 2779-2793
Andersen J-T et al (2010) The Journal of Biological Chemistry 285: 4826-4836
Gaboriaud, C. et al (2003) The Journal of Biological Chemistry 278: 46974-82
Hosszu, K.K. et al (2012) Blood 120: 1228-1236
Mammen, M. et al (1998) Angew. Chem. Int. Edit. 37: 2755-2794
Inoue, Y. et al. (2007) J. Immunol. 179, 764-774
Siragam, V. et al. J. Clin. Invest. 115: 155-60 (2005)
Siragam, V. et al. Nat. Med. 12: 688-92 (2006)
Pleass RJ & Woof JM. Trends in Parasitol 17: 545-51 (2001).

## Claims

1. A polymeric protein consisting of six polypeptide monomer units for use in a method of treating an autoimmune or inflammatory disease in a mammalian subject,
wherein the method comprises administering to the mammalian subject an effective amount of the polymeric protein;
wherein each polypeptide monomer unit consists of an Fc receptor binding portion consisting of two immunoglobulin G heavy chain constant regions;
wherein each immunoglobulin G heavy chain constant region comprises a cysteine residue which is linked via a disulfide bond to a cysteine residue of an immunoglobulin G heavy chain constant region of an adjacent polypeptide monomer unit; and
wherein a tailpiece region is fused to each of the two immunoglobulin G heavy chain constant regions, wherein the tailpiece regions facilitate the assembly of the monomer units into a polymer.

2. The polymeric protein for the use of Claim 1 wherein the tailpiece region is an IgM or IgA tailpiece, or fragment or variant thereof.

3. The polymeric protein for the use of Claim 1 wherein each of the immunoglobulin G heavy chain constant regions comprises an amino acid sequence having at least 90% sequence identity to a native human immunoglobulin G1 heavy chain constant region.

4. The polymeric protein for the use of Claim 1 wherein each of the immunoglobulin G heavy chain constant regions comprises an amino acid sequence which comprises a cysteine residue at position 309 according to the EU numbering system, and preferably also a leucine residue at position 310.

5. The polymeric protein for the use of Claim 1 wherein the autoimmune or inflammatory disease is treatable with intravenous immunoglobulin (IVIG).

6. The polymeric protein for the use of Claim 1 wherein the autoimmune or inflammatory disease is an autoimmune cytopenia, idiopathic thrombocytopenic purpura, rheumatoid arthritis, systemic lupus erythematosus, asthma, Kawasaki disease, Guillain-Barre syndrome, Stevens-Johnson syndrome, Crohn's colitis, diabetes, chronic inflammatory demyelinating polyneuropathy myasthenia gravis, anti-Factor VIII autoimmune disease, dermatomyositis, vasculitis, and uveitis or Alzheimer's disease.

7. The polymeric protein for the use of Claim 1 wherein, in each polypeptide monomer unit, a polypeptide linker links the two immunoglobulin G heavy chain constant regions as a single chain Fc.

## Patentansprüche

1. Polymeres Protein, bestehend aus sechs Polypeptid-Monomereinheiten zur Verwendung in einem Verfahren zur Behandlung einer Autoimmun- oder inflammatorischen Erkrankung in einem Säugetiersubjekt, wobei das Verfahren das Verabreichen einer wirksamen Menge des polymeren Proteins an das Säugetiersubjekt umfasst;
wobei jede Polypeptid-Monomereinheit aus einer Fc-Rezeptorbindungsregion besteht, die aus zwei konstanten Regionen von schwerkettigem Immunglobulin-G besteht;
wobei jede konstante Region von schwerkettigem Immunglobulin-G einen Cysteinrest aufweist, der über eine Disulfidbindung mit einer konstanten Region von schwerkettigem Immunglobulin-G einer benachbarten Polypeptid-Monomereinheit verbunden ist; und
wobei eine Endregion an jede der konstanten Regionen von schwerkettigem Immunglobulin-G fusioniert ist, wobei die Endregionen die Assemblierung der Monomereinheiten zu einem Monomer ermöglichen.

2. Polymeres Protein nach Anspruch 1, wobei die Endregion ein IgM- oder IgA-Ende oder ein Fragment oder eine Variante davon ist.

3. Polymeres Protein nach Anspruch 1, wobei jede der konstanten Regionen von schwerkettigem Immunglobulin-G eine Aminosäuresequenz umfasst, mit einer Sequenzübereinstimmung von mindestens 90% mit einer nativen menschlichen konstanten Region von schwerkettigem Immunglobulin-G1.

4. Polymeres Protein nach Anspruch 1, wobei jede der konstanten Regionen von schwerkettigem Immunglobulin-G eine Aminosäuresequenz umfasst, die einen Cysteinrest an Position 309 des EU-Nummerierungsschemas und vorzugsweise eine Leucinrest an Position 310 umfasst.

5. Polymeres Protein nach Anspruch 1, wobei die Autoimmun- oder inflammatorische Erkrankung mit intravenösem Immunglobulin (IVIG) behandelbar ist.

6. Polymeres Protein nach Anspruch 1, wobei die Autoimmun- oder inflammatorische Erkrankung autoimmune Zytopenie, idiopathische thrombozytopenische Purpura, rheumatoide Arthritis, Systematische Lupus Erythematodes, Asthma, Kawasaki-Syndrom, Guillain-Barré-Syndrom, Stevens-Johnson-Syndrom, Crohnsche Colitis, Diabetes, chronisch inflammatorische demyelinisierende Polyneuropathie Myasthenia gravis, Antifaktor-VIII-Autoimmunerkrankung, Dermatomyositis, Vasculitis und Uveitis oder Alzheimer-Krankheit ist.

7. Polymeres Protein nach Anspruch 1, wobei in jeder Polypeptid-Monomereinheit ein Polypeptid-Linker die zwei konstanten Regionen von schwerkettigem Immunglobulin-G als eine Kette Fc verbindet.

## Revendications

1. Protéine polymère comprenant six unités monomères polypeptidiques destinée à être utilisée dans un procédé de traitement d'une maladie auto-immune ou inflammatoire chez un sujet mammifère, le procédé comprenant l'étape consistant à administrer au sujet mammifère une quantité efficace de la protéine polymère ;
chaque unité monomère polypeptidique comprenant une partie de liaison de récepteur Fc comprenant deux régions constantes de chaîne lourde d'immunoglobuline G;
chaque région constante de chaîne lourde d'immunoglobuline G comprenant un résidu cystéine qui est lié par l'intermédiaire d'une liaison disulfure à un résidu cystéine d'une région constante de chaîne lourde d'immunoglobuline G d'une unité monomère polypeptidique adjacente ; et
une région de raccordement étant fusionnée à chacune des deux régions constantes de chaîne lourde d'immunoglobuline G, les régions de queue facilitant l'assemblage des unités monomères en un polymère.

2. Protéine polymère destinée à être utilisée selon la revendication 1, la région de queue étant une queue IgM ou IgA, ou un fragment ou une variante de celle-ci.

3. Protéine polymère destinée à être utilisée selon la revendication 1, chacune des régions constantes de chaîne lourde d'immunoglobuline G comprenant une séquence d'acides aminés ayant au moins 90 % d'identité de séquence avec une région constante de chaîne lourde d'immunoglobuline G1 humaine native.

4. Protéine polymère destinée à être utilisée selon la revendication 1, chacune des régions constantes de chaîne lourde d'immunoglobuline G comprenant une séquence d'acides aminés qui comprend un résidu cystéine en position 309 selon le système de numérotation UE, et de préférence aussi un résidu leucine en position 310.

5. Protéine polymère destinée à être utilisée selon la revendication 1, la maladie auto-immune ou inflammatoire pouvant être traitée par immunoglobuline intraveineuse (IVIG).

6. Protéine polymère destinée à être utilisée selon la revendication 1, la maladie auto-immune ou inflammatoire étant la cytopénie auto-immune, le purpura thrombocytopénique idiopathique, la polyarthrite rhumatoïde, le lupus érythémateux systémique, l'asthme, la maladie de Kawasaki, le syndrome de Guillain-Barré, le syndrome de Stevens-Johnson, la colite de Crohn, le diabète, la myasthénie grave chronique inflammatoire polyneuropathique démyélinisante, la maladie auto-immune anti-Factor VIII, la dermatomyosite, la vasculite et l'uvéite ou la maladie d'Alzheimer.

7. Protéine polymère destinée à être utilisée selon la revendication 1, dans chaque unité monomère polypeptidique, un agent de liaison de polypeptide liant les deux régions constantes de chaîne lourde d'immunoglobuline G en tant que chaîne unique Fc.
